# EUROPÄISCHE PATENTSCHRIFT

(11) **EP 3 474 833 B1**
(45) Veröffentlichungstag und Bekanntmachung des Hinweises auf die Patenterteilung: **19.08.2026**
(21) Anmeldenummer: 16732290.8
(22) Anmeldetag: 24.06.2016
(51) Int. Cl.: A61K 31/015, A61K 31/185, A61P 3/04, A61P 25/24, A61K 31/215

(54) **CINNAMYLALKOHOLDERIVATE ZUR VERWENDUNG IN DER REDUZIERUNG DES APPETITS UND ZUR VERMITTLUNG EINES GEFÜHLS VON SÄTTIGUNG**
CINNAMYL ALCOHOL DERIVATIVE FOR USE IN REDUCING APPETITE AND FOR GENERATING THE FEELING OF BEING FULL
DÉRIVÉS DE L'ALCOOL CINNAMIQUE UTILISÉS POUR RÉDUIRE L'APPÉTIT ET POUR PROCURER UNE SENSATION DE SATIÉTÉ

(43) Veröffentlichungstag der Anmeldung: 01.05.2019
(73) Patentinhaber: Symrise AG, 37603 Holzminden (DE)
(72) Erfinder: SOMOZA, Veronika, 3400 Weidling (AT); LIEDER, Barbara, 1230 Wien (AT); GEISSLER, Katrin, 37574 Einbeck (DE); HANS, Joachim, 37603 Holzminden (DE); LANGER, Kathrin, 37586 Dassel (DE); LEY, Jakob, Peter, 37603 Holzminden (DE); WIDDER, Sabine, 37603 Holzminden (DE)
(74) Vertreter: Eisenführ Speiser
(86) Internationale Anmeldenummer: PCT/EP2016/064742
(87) Internationale Veröffentlichungsnummer: WO 2017/220168

(56) Entgegenhaltungen:
- EP-A1- 2 614 727
- EP-A1- 2 918 270
- WO-A1-99/53935
- CN-A- 1 320 438
- CN-A- 1 994 309
- DE-U1- 20 206 521

## Beschreibung

Die vorliegende Erfindung betrifft bestimmte Cinnamylalkoholderivate zur Anwendung in einem therapeutischen Verfahren (a) zur Reduzierung des Appetits und/oder (d) zur Reduzierung des Körpergewichts und/oder (e) zur Stimmungsaufhellung. Zudem betrifft die Erfindung Zubereitungen, insbesondere oral konsumierbare Produkte, umfassend die erfindungsgemäßen Cinnamylalkoholderivate zur Anwendung in entsprechenden therapeutischen Verfahren.

Die Erfindung betrifft ferner die nicht-therapeutische Verwendung der erfindungsgemäßen Cinnamylalkoholderivate als (a) Mittel zur Reduzierung des Appetits und/oder (b) Mittel zur Vermittlung eines Gefühls von Sättigung und/oder als (c) Mittel zur Reduzierung der Energieaufnahme und/oder (d) Mittel zur Reduzierung des Körpergewichts und/oder als (e) Stimmungsaufheller.

Schließlich betrifft die Erfindung auch nicht-therapeutische Verfahren zur (i) Reduzierung des Appetits und/oder (ii) Vermittlung eines Gefühls von Sättigung und/oder (iii) Reduzierung der Energieaufnahme und/oder (iv) Reduzierung des Körpergewichts und/oder (v) Aufhellung der Stimmung umfassend den Schritt des Verabreichens der erfindungsgemäßen Cinnamylalkoholderivate an ein Individuum.

Weitere Aspekte der vorliegenden Erfindung und bevorzugte Ausgestaltungen ergeben sich aus der nachfolgenden Beschreibung sowie insbesondere den beigefügten Patentansprüchen.

Das durch mangelnde Bewegung und/oder zu hohe Nahrungsaufnahme verursachte häufige Auftreten von andauerndem Übergewicht kann zu chronischen Krankheitsbildern wie Fettleibigkeit, Insulinresistenz, Lipidstoffwechselstörungen und/oder Bluthochdruck, deren schwerwiegenden Folgeerkrankungen Diabetes Typ II, Arteriosklerose, Herz- oder Hirninfarkt und damit letztendlich zum verfrühten Tod führen. Insbesondere ein hoher Gehalt an leicht verstoffwechselbaren Kohlenhydraten, Proteinen und vor allem Fetten in der Nahrung führt zur Bildung von Fettdepots und kann schließlich zu den genannten Problemen stark beitragen. Um die Aufnahme dieser hedonisch oft sehr bevorzugten Nahrungsmittelbestandteile, vor allem der Fette und süßen Kohlenhydrate (Zucker), zu begrenzen, werden oft deren Gehalte in kalorienreduzierten Lebensmitteln, den sogenannten "Light-Produkten", stark gesenkt und durch Ersatzstoffe (wie beispielsweise Verdicker für Fette, nicht-kalorische Süßstoffe statt Zucker) ausgetauscht.

Beim Konsum von "Light-Produkten" kann es bei einem Produkt, dass aufgrund geschickter Formulierung einen vergleichbaren hedonischen Wert wie das hochenergetische Originalprodukt besitzt, oft vorkommen, dass es in größeren Mengen konsumiert wird und es dann im ungünstigsten Fall sogar zu einer erhöhten Aufnahme kalorisch relevanter Nahrungsmittelbestandteile kommt und somit das Ziel verfehlt wird, die aufgenommene Kalorienmenge abzusenken.

Um einer erhöhten Aufnahme kalorisch relevanter Nahrungsmittelbestandteile entgegenzuwirken, besteht schon länger der Wunsch, Nahrungsbestandteile, insbesondere als sicher bewertete, bereits zugelassene und allgemein akzeptierte Aromastoffe zu finden, die das Hungergefühl und den natürlichen Appetit reduzieren und/oder das Sättigungsgefühl entsprechend erhöhen können. Es ist bekannt, dass durch Erhöhung der Serotonin-Ausschüttung in bestimmten Hirnarealen bei gleichzeitiger Exposition mit Nährstoffen durch die aufgenommenen oral konsumierbaren Produkte (insbesondere Lebensmittel) sowie durch Induktion von Leptin-Rezeptor- und Serotonin-Rezeptor-Proteinen der Appetit negativ und die Sättigung positiv beeinflusst werden können.

Neben Auswirkungen auf den Appetit und auf die Sättigung, kann auch die Stimmung des Konsumenten positiv beeinflusst werden. So sind bereits Lebensmittel bekannt, die eine stimmungsaufhellende Wirkung besitzen. Sofern Serotonin in ausreichenden Mengen im Hirn vorhanden ist, vermittelt es eine positive Stimmung, eine gute Konzentrationsfähigkeit und Optimismus. Niedrige Serotoninspiegel können hingegen zu Reizbarkeit, Schlafstörungen, mangelnder Konzentrationsfähigkeit und Depressionen führen.

Eine Aufnahme von Serotonin und Dopamin über die Nahrung kann die Serotonin- bzw. Dopaminkonzentration im Blut erhöhen und mit einiger Wahrscheinlichkeit auch in Wechselwirkungen mit Rezeptoren treten, um auch die Serotonin- bzw. Dopaminkonzentration im Gehirn zu erhöhen.

In EP 2 614 727 wird Nonivamid als ein äußerst wirksamer Aromastoff beschrieben, der die gewünschten Wirkungen zeigt. Allerdings ist durch die intrinsische Schärfe des Stoffs die Einsetzbarkeit insbesondere in milder aromatisierten Endanwendungen problematisch.

CN1320438A offenbart die Verwendung von u.a. Zimtalkohol für die Behandlung von Diabetes, Hyperlipidämie, eines erhöhten Cholesterinspiegels und Übergewicht.

CN1994309A betrifft die Verwendung verschiedener Substanzen für die Behandlung von Depression und Angstzuständen. Unter den eingesetzten Substanzen befindet sich auch Cinnamylacetat und Zimtalkohol.

Es war daher Aufgabe der vorliegenden Erfindung, Verbindungen bereitzustellen, die einen Beitrag zur Reduzierung des Appetits und/oder zur Vermittlung eines Gefühls von Sättigung und/oder zur Reduzierung der Energieaufnahmen und/oder zur Reduzierung des Körpergewichts und/oder zur Stimmungsaufhellung leisten können und dabei in einer Vielzahlt von Produkten einsetzbar sind.

Insbesondere war es Aufgabe der vorliegenden Erfindung, solche Verbindungen bereitzustellen, die nicht toxisch sind und die Körpergesundheit positiv beeinflussen.

Ferner war es Aufgabe der vorliegenden Erfindung, solche Verbindungen bereitzustellen, die bereits in geringen Mengen die oben genannten Wirkungen haben und keine unangenehmen sensorischen Noten oder einen Nebengeschmack in Produkten verursachen.

Diese Aufgaben werden erfindungsgemäß gelöst durch ein Cinnamylalkoholderivat der Formel I oder Mischung aus zwei oder mehr Cinnamylalkoholderivaten der Formel I 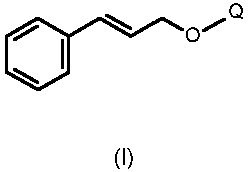
wobei die Doppelbindung eine E- oder Z-Konfiguration aufweisen kann oder eine beliebige Mischung von E- und Z-Konfigurationen vorliegen kann, und wobei Q ein Acylrest der Formel II 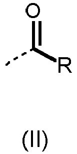
ist, wobei R Wasserstoff oder Methyl oder ein gesättigter oder ungesättigter, linearer oder verzweigter oder cylischer aliphatischer Rest mit 2 bis 6 Kohlenstoffatomen oder Phenyl oder Phenylmethyl ist,
zur Anwendung in einem therapeutischen Verfahren (a) zur Reduzierung des Appetits und/oder (d) zur Reduzierung des Körpergewichts und/oder (e) zur Stimmungsaufhellung, oder
Cinnamylalkoholderivat der Formel I, wobei Q ein Acylrest der Formel II und R Methyl ist,
zur Anwendung in einem therapeutischen Verfahren (a) zur Reduzierung des Appetits und/oder (d) zur Reduzierung des Körpergewichts.

In einer bevorzugten Ausführungsform werden die Cinnamylalkoholderivate der Formel I zur Verwendung bei der Behandlung von Adipositas eingesetzt.

Gemäß einer bevorzugten Ausführungsform, ist bei dem Cinnamylalkoholderivat der Formel I oder der Mischung aus zwei oder mehr Cinnamylalkoholderivaten der Formel I zur Anwendung wie oben beschrieben R (im Falle des Vorliegens einer Mischung jeweils unabhängig von einander) ausgewählt aus der Gruppe bestehend aus Wasserstoff, Methyl, Ethyl, Propyl, 2-Propyl, 1-Butyl, 2-Butyl, 2-Methyl-1-propyl, 1-Pentyl, 2-Pentyl, 3-Pentyl, 2-Methyl-1-butyl, 2-Methyl-2-butyl, 2-Methyl-3-butyl, 3-Methyl-1-butyl, 1-Hexyl, 2-Hexyl, 3-Hexyl, 2-Methyl-1-pentyl, 2-Methyl-2-pentyl, 2-Methyl-3-pentyl, 2-Methyl-4-pentyl, 3-Methyl-1-pentyl, 3-Methyl-2-pentyl, 3-Methyl-3-pentyl, Vinyl, E- und Z- 1-Propen-1-yl, 2-Propen-1-yl, E- und Z- 2-Buten-2-yl, 3-Buten-2-yl, E- und Z-Prenyl, E- und Z-Isoprenyl, Phenyl und Phenylmethyl.

Insbesondere erfindungsgemäß bevorzugt ist ein bzw. sind Cinnamylalkoholderivat(e) der Formel I zur Anwendung wie oben beschrieben, das/die ausgewählt ist/sind aus der Gruppe bestehend aus: 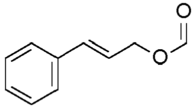
Cinnamylformiat (Verbindung 2, FEMA# 2299) 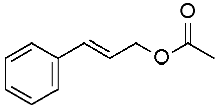
Cinnamylacetat (Verbindung 3, FEMA# 2293) 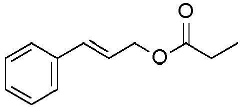
Cinnamylpropionat (Verbindung 4, FEMA# 2301) 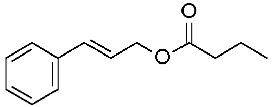
Cinnamylbutyrat (Verbindung 5, FEMA# 2296) 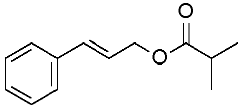
Cinnamylisobutyrat (Verbindung 6, FEMA# 2297) 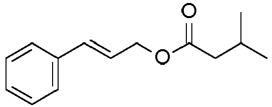
Cinnamylisovalerianat (Verbindung 7, FEMA# 2302) 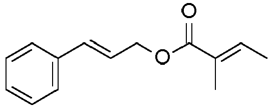
Cinnamyltiglinat (Verbindung 8, EC Aromastoffliste 09.339) 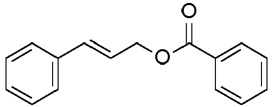
Cinnamylbenzoat (Verbindung 9, FEMA# 4703) 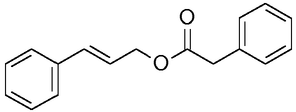
Cinnamylphenylacetat (Verbindung 10, FEMA# 2300) 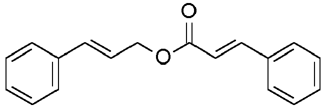
Cinnamylcinnamat (Verbindung 11, FEMA# 2298)

Gemäß einer weiteren bevorzugten Ausführungsform liegt das Cinnamylalkoholderivat der Formel I oder liegen die Cinnamylalkoholderivate der Formel I in der Mischung zur Anwendung wie oben beschrieben, (jeweils) zu mehr als 50 %, bevorzugt zu mehr als 90 %, besonders bevorzugt zu mehr als 95 % in der E-Konfiguration vor.

Ein weiterer Aspekt der vorliegenden Erfindung betrifft eine Zubereitung umfassend ein Cinnamylalkoholderivat der Formel I oder eine Mischung aus zwei oder mehr Cinnamylalkoholderivaten der Formel I wie oben beschrieben zur Anwendung in einem therapeutischen Verfahren wie oben beschrieben.

Die Cinnamylalkoholderivate der Formel I werden bevorzugt in Zubereitungen eingearbeitet, welche bevorzugt oral konsumierbare Produkte sind. Eine solche Zubereitung umfasst vorzugsweise mindestens ein, zwei, drei oder vier Cinnamylalkoholderivate der Formel I, vorzugsweise Verbindungen 2 bis 11.

Gemäß einer bevorzugten Ausgestaltung liegt die Gesamtkonzentration der Cinnamylalkoholderivate der Formel I in der Zubereitung zur Anwendung wie oben beschrieben bei 20 mg/kg oder weniger, bevorzugt bei 5 mg/kg oder weniger, besonders bevorzugt bei 2 mg/kg oder weniger, jeweils bezogen auf die Gesamtmasse der Zubereitung.

Überraschenderweise hat sich gezeigt, dass die erfindungsgemäß anzuwendenden Cinnamylalkoholderivate der Formel I in der Lage sind, bereits in einem Konzentrationsbereich von 10 µM (ca. 1 mg/kg bis ca. 3 mg/kg) die Serotonin-Freisetzung in Neuronen auf bis zu ca. 130 % der Positivkontrolle (Nonivamid, EP 2 614 727 ) anzuregen.

Die jeweiligen Konzentrationen der aromatischen Cinnamylalkoholderivate sind im Vergleich zu typischen Einsatzkonzentrationen, um einen Geschmackseffekt zu erzielen, gering. Es ist zwar mit Aromaeffekten zu rechnen, diese sollten aber auch in mild aromatisierten oral konsumierbaren Produkten nicht bestimmend sein.

Vorzugsweise weisen die erfindungsgemäß anzuwendenden Zubereitungen mindestens 1, 2, 3, 4, 5, 6, 7, 8, 9 oder 10 Cinnamylalkoholderivate der Formel I auf, welche bevorzugt ausgewählt sind aus der Gruppe der Verbindung 2, Verbindung 3, Verbindung 4, Verbindung 5, Verbindung 6, Verbindung 7, Verbindung 8, Verbindung 9, Verbindung 10 oder Verbindung 11. Ebenfalls ist es vorteilhaft, wenn alle 10 Verbindungen in einer solchen Zubereitung vorliegen. Bei der Bestimmung der Gesamtkonzentration der Cinnamylalkoholderivate in einer Zubereitung ist die Summe der Konzentrationen der einzelnen Verbindungen gemeint.

In einer besonders bevorzugten erfindungsgemäß anzuwendenden Zubereitung zur Anwendung wie oben beschreiben, enthält die Zubereitung weitere Stoffe, bei denen es sich vorzugsweise um sprühgetrocknete Stoffe handelt, die zum Verzehr geeignete Bestandteile, feste Trägerstoffe und optional bestimmte Aromastoffe bzw. Aromakompositionen umfassen.

In weiteren besonders bevorzugten erfindungsgemäß anzuwendenden Zubereitungen zur Anwendung wie oben beschrieben, handelt es sich bei den weiteren Stoffen um zum Verzehr geeignete feste Trägerstoffe.

Eine bevorzugte Zubereitung zur Anwendung wie oben beschrieben umfasst mindesten einen festen Trägerstoff und vorzugsweise mindestens einen weiteren Aromastoff. Solche Zubereitungen sind vorzugsweise sprühgetrocknet.

Vorteilhafte Trägerstoffe in diesen bevorzugten erfindungsgemäß anzuwendenden (vorzugsweise sprühgetrockneten) Zubereitungen sind Siliciumdioxid (Kieselsäure, Kieselgel), Kohlenhydrate und/oder Kohlenhydratpolymere (Polysaccharide), Cyclodextrine, Stärken, abgebaute Stärken (Stärkehydrolysate), chemisch oder physikalisch modifizierte Stärken, modifizierte Cellulosen, Gummi Arabicum, Ghatti-Gummi, Traganth, Karaya, Carrageenan, Guarkernmehl, Johannisbrotkernmehl, Alginate, Pektin, Inulin oder Xanthan Gum zu nennen. Bevorzugte Stärkehydrolysate sind Maltodextrine und Dextrine.

Bevorzugte Trägerstoffe sind Siliciumdioxid, Gummi Arabicum und Maltodextrine, wobei hier wiederum Maltodextrine mit DE-Werten im Bereich 5 bis 20 bevorzugt sind. Es ist dabei unerheblich, welche Pflanze ursprünglich die Stärke zur Herstellung der Stärkehydrolysate geliefert hat. Geeignet und leicht verfügbar sind Mais-basierende Stärken sowie Stärken aus Tapioka, Reis, Weizen oder Kartoffeln. Die Trägerstoffe können dabei auch als Fließhilfsmittel fungieren, wie beispielsweise Siliciumdioxid.

Die erfindungsgemäß anzuwendenden Zubereitungen, die neben dem Cinnamylalkoholderivat oder mehreren Cinnamylalkoholderivaten noch einen oder mehrere feste Trägerstoffe umfassen, können beispielsweise durch mechanische Mischvorgänge, wobei gleichzeitig auch eine Zerkleinerung der Partikel erfolgen kann, oder mittels Sprühtrocknung hergestellt werden. Bevorzugt sind erfindungsgemäß anzuwendende Zusammensetzungen, die feste Trägerstoffe umfassen und mittels Sprühtrocknung hergestellt sind; hinsichtlich der Sprühtrocknung wird verwiesen auf US 3,159,585, US 3,971,852, US 4,532,145 oder US 5,124,162.

Bevorzugte erfindungsgemäß anzuwendende, Trägerstoffe umfassende Zubereitungen, die mittels Sprühtrocknung hergestellt wurden, besitzen eine mittlere Partikelgröße im Bereich von 30 bis 300 µm und bevorzugt eine Restfeuchte von kleiner oder gleich 5 Gew.-%.

Der oder zumindest einer der weiteren Aromastoffe in der Zubereitung ist vorzugsweise eine sensorisch wirksame Komponente und wird bevorzugt eingesetzt in einer Konzentration, die größer ist als sein Reizschwellenwert.

Beispiele für weitere Aromastoffe, die Bestandteil der Zubereitung sein können, finden sich z.B. in H. Surburg, J. Panten, Common Fragrance and Flavor Materials, 5th. Ed., Wiley-VCH, Weinheim 2006.

Aromastoffe können in Form von Aromakompositionen eingesetzt werden, die wiederum in Form von Reaktionsaromen (Maillard-Produkte) und/oder Extrakten bzw. ätherischen Ölen von Pflanzen oder Pflanzenteilen bzw. Fraktionen davon eingesetzt werden können.

Ebenso ist eine Kombination mit Capsaicinoiden, insbesondere mit Nonivamid (N-Nonanoylvanillylamin), welche ebenfalls zur Reduzierung des Appetits und/oder zur Vermittlung eines Gefühls von Sättigung und/oder zur Aufhellung der Stimmung eingesetzt werden kann, möglich.

Gemäß einer weiteren bevorzugten Ausführung enthält die Zubereitung zur Anwendung wie oben beschrieben daher zusätzlich Nonivamid.

Nonivamid (N-Nonanoylvanillylamin) und die erfindungsgemäß anzuwendenden Cinnamylalkoholderivate der Formel I weisen gleichwertige Aktivitäten zur Reduzierung des Appetits, Reduzierung der kalorischen Aufnahme und Reduktion des Körpergewichtes, und/oder zur Vermittlung eines Gefühls von Sättigung und/oder zur Aufhellung der Stimmung, auf.

Die Kombination aus Nonivamid (N-Nonanoylvanillylamin) und den erfindungsgemäß anzuwendenden Cinnamylalkoholderivaten der Formel I, insbesondere den Verbindungen 2 bis 11, einzeln oder im Gemisch, ist daher besonders vorteilhaft, da insbesondere die erfindungsgemäß anzuwendenden Cinnamylalkoholderivate bei der oben genannten Dosierung einen gegenüber Nonivamid sensorisch niedrigeren Wert bei gleichbleibender erfindungsgemäßer Wirkung aufweisen. Dies ist insbesondere vorteilhaft, da die Cinnamylalkoholderivate, insbesondere Verbindungen 2 bis 11, in Zubereitungen, wie oral konsumierbaren Produkten, in einer höheren Menge einsetzbar sind oder einen Teil des Nonivamids ersetzen können, ohne den Geschmack negativ zu beeinflussen bzw. sogar den durch Nonivamid ausgelösten Geschmackswert unter deren jeweiligen Schwellenwert senken können.

In einer besonders bevorzugten erfindungsgemäß anzuwendenden Zubereitung zur Anwendung in einem therapeutischen Verfahren wie oben beschrieben, umfasst die Zubereitung Capsaicin und/oder N-Nonanoylvanillylamin (Nonivamid).

Bei allen Ausführungsformen sind Einzelstoffe oder Mischungen der Cinnamylalkoholderivate der Formel I bevorzugt, die nur einen unterschwelligen Aromawert in der Einsatzkonzentration zeigen, d.h. bevorzugt werden die Verbindungen um den Erkennnungsschwellenwert herum oder niedriger eingesetzt (bezogen auf die verzehrfertige Zubereitung). Das gilt ebenfalls auch für die Verbindung Nonivamid (N-Nonanoylvanillylamin), wenn diese in einer solchen Zubereitung vorliegt.

Demgemäß ist eine bevorzugte Ausführungsform eine Zubereitung zur Anwendung in einem Verfahren wie oben beschrieben, wobei die Menge an Cinnamylalkoholderivaten und/oder Nonivamid (N-Nonanoylvanillylamin) in einer Konzentration eingesetzt wird, die nur einen unterschwelligen Aromawert in der Einsatzkonzentration zeigen.

Besonders bevorzugt ist eine Zubereitung, insbesondere ein oral konsumierbares Produkt wie oben beschrieben, zur Anwendung wie oben beschrieben, wobei die Cinnamylalkoholderivate der Formel I in einer Konzentration von 20 mg/kg oder weniger, bevorzugt 5 mg/kg oder weniger, besonders bevorzugt 2 mg/kg oder weniger und in einer Konzentration von 0.0001 mg/kg oder mehr, vorzugsweise 0.001 mg/kg oder mehr, besonders bevorzugt 0.005 mg/kg oder mehr vorliegen, jeweils bezogen auf die Gesamtmasse der Zubereitung.

Besonders vorteilhaft ist auch eine Zubereitung, insbesondere ein oral konsumierbares Produkt, zur Anwendung wie oben beschrieben, wobei die Zubereitung zusätzlich essbare tierische oder vegane Proteine in Form von Isolaten, Fraktionen, partiellen oder vollständigen Hydrolysaten oder durch physikalischchemische Prozesse oder fermentative oder enzymatische Behandlung der Proteine hergestellte Zwischenprodukte und optional einen oder mehrere weitere, die Sättigung beeinflussenden Zusatz- oder Aromastoff, vorzugsweise nichtverdauliche Kohlenhydrate wie Pektine, beta-Glucane enthält. Vorzugsweise ist der Gehalt an solchen Proteinen bzw. Proteinprodukten höher als in herkömmlichen Zubereitungen, wodurch noch schneller ein Sättigungsgefühl erreicht wird.

Quellen für essbare tierische oder vegane Proteine können ausgewählt werden aus: Fleischprodukten (Säugetiere, Vögel, Reptilien, Amphibien, Fisch), Krebse, Schalentiere, Muscheln, Weichtiere, Insekten, Eier, Getreideproteine, Milch, Algen-, Weizen-, Gerste-, Raps-, Sonnenblumen-, Reis-, Kartoffel-, Mais-, Soja- , Bohnen-, Erbsen-, Linsen-, Lupinen-, Erdnuss-, Alfalfa-Proteinisolaten, wobei weitere Proteine aus essbaren Pflanzen, die hier nicht genannt sind, auch verwendet werden können.

Bevorzugt ist eine erfindungsgemäß anzuwendende Zubereitung ein oral konsumierbares Produkt oder Bestandteil eines oral konsumierbaren Produktes. Insbesondere bevorzugt ist eine solche Zubereitung ausgewählt aus der Gruppe bestehend aus flüssigen oder festen Lebensmitteln, Halbfertigwaren, Futtermitteln, kosmetischen Anwendungen und Arzneimitteln.

Gemäß einem bevorzugten Aspekt betrifft die vorliegende Erfindung daher eine Zubereitung zu Anwendung wie oben beschrieben, welche ein oral konsumierbares Produkt ist.

Vorzugsweise werden die genannten erfindungsgemäß anzuwendenden Cinnamylalkoholderivate zur erfindungsgemäßen Anwendung in einem therapeutischen Verfahren eingesetzt, wobei die erfindungsgemäßen Cinnamylalkoholderivate vorzugsweise alleine oder als Gemisch Bestandteil eines oral konsumierbaren Produktes (insbesondere eines Lebensmittels, Futtermittels oder Arzneimittels) eingesetzt werden, wobei die erfindungsgemäß anzuwendenden Cinnamylalkoholderivate der Formel I in einer Konzentration von 20 mg/kg oder weniger vorliegen, bezogen auf die Gesamtmasse des oral konsumierbaren Produktes.

Bevorzugt ist ein erfindungsgemäß anzuwendendes oral konsumierbares Produkt ausgewählt aus der Gruppe bestehend aus Lebensmitteln (insbesondere flüssige oder feste, einschließlich Halbfertigwaren), Futtermitteln und Arzneimitteln (pharmazeutische Zubereitungen).

Im Rahmen des vorliegenden Textes umfasst der Begriff "Lebensmittel" eine Vielzahl von Produkten. Unter den Begriff Lebensmittel fallen insbesondere Produkte, wie sie weiter unten im Zusammenhang mit erfindungsgemäß anzuwendenden Lebensmitteln diskutiert werden.

Der Begriff "Lebensmittel" umfasst insbesondere Produkte, die gemäß der VERORDNUNG (EG) Nr. 178/2002 DES EUROPÄISCHEN PARLAMENTS UND DES RATES vom 28. Januar 2002 Lebensmittel sind. Gemäß dieser Verordnung sind "Lebensmittel" alle Stoffe oder Erzeugnisse, die dazu bestimmt sind oder von denen nach vernünftigem Ermessen erwartet werden kann, dass sie in verarbeitetem, teilweise verarbeitetem oder unverarbeitetem Zustand von Menschen aufgenommen werden. Zu "Lebensmitteln" zählen auch Getränke, Kaugummi sowie alle Stoffe - einschließlich Wasser -, die dem Lebensmittel bei seiner Herstellung oder Ver- oder Bearbeitung absichtlich zugesetzt werden.

Der Begriff "Futtermittel" umfasst im Rahmen des vorliegenden Textes alle Formen von Tiernahrung. Eine Vielzahl der nachstehend genannten Lebensmittel kann auch als Futtermittel eingesetzt werden.

Der Begriff "Arzneimittel" umfasst im Rahmen des vorliegenden Textes Stoffe oder Stoffzusammensetzungen, die als Mittel mit Eigenschaften zur Heilung oder zur Verhütung menschlicher oder tierischer Krankheiten bestimmt sind oder aber im oder am menschlichen oder tierischen Körper verwendet oder einem Menschen bzw. Tier verabreicht werden können, um entweder die menschlichen bzw. tierischen physiologischen Funktionen durch eine pharmakologische, immunologische oder metabolische Wirkung wiederherzustellen, zu korrigieren oder zu beeinflussen oder eine medizinische Diagnose zu erstellen. Arzneimittel können im Einzelfall zu nicht-therapeutischen, insbesondere kosmetischen Zwecken eingesetzt werden.

Es versteht sich, dass Lebensmittel oder Futtermittel durch den Zusatz von Stoffen oder Stoffzusammensetzungen, die als Mittel mit Eigenschaften zur Heilung oder zur Verhütung menschlicher oder tierischer Krankheiten bestimmt sind, in entsprechende Arzneimittel überführt werden können.

Die vorliegende Erfindung betrifft auch erfindungsgemäß anzuwendende oral konsumierbare Produkte, umfassend mindestens 1, 2, 3, 4, 5, 6, 7, 8, 9 oder 10 Cinnamylalkoholderivate der Formel I. Vorzugsweise umfasst ein solches oral konsumierbares Produkt mindestens 1, mindestens 2, mindestens 3, mindestens 4 Verbindungen ausgewählt aus der Gruppe der Verbindung 4, Verbindung 6, Verbindung 7 oder Verbindung 11.

Vorzugsweise liegen die erfindungsgemäß einzusetzenden Cinnamylalkoholderivate zu mehr als 50 %, besonders bevorzugt zu mehr als 90 %, insbesondere bevorzugt zu mehr als 95 % in der E-Konfiguration vor: Dabei bezieht sich die Angabe der Konfiguration jeweils auf den Cinnamylrest.

Um eine Reduktion des Körpergewichtes beim Konsumenten zu unterstützen, hat es sich als sinnvoll erwiesen, die potentielle kalorische Aufnahme zu begrenzen. Dies kann zum einen dadurch erfolgen, dass der erfindungsgemäße Einsatz des Cinnamylalkoholderivates oder einer Mischung der Cinnamylalkoholderivate in oral konsumierbaren Produkten (insbesondere Lebensmitteln, Futtermitteln und Arzneimitteln) ein Gefühl von Sättigung vermittelt und gleichzeitig den Appetit reduziert, sowie zusätzlich die Absorption von energieliefernden Nährstoffen aus dem Darm verringert. Dadurch wird der Konsument nicht nur dazu veranlasst, den Verzehr von energiehaltigen Produkten zu begrenzen, sondern ihm steht von der oral aufgenommenen Energiemenge durch eine verringerte Absorption auch weniger Nahrungsenergie zur Verfügung. Neben der durch die Cinnamylalkoholderivate verursachten geringeren Aufnahme und Akkumulation von Lipiden lässt die sich kalorische Aufnahme zusätzlich reduzieren, wenn erfindungsgemäß anzuwendende oral konsumierbare Produkte (insbesondere Lebensmittel, Futtermittel bzw. Arzneimittel) zum Verzehr angeboten werden, die ihrerseits bereits eine geringe Energiedichte aufweisen.

Ein bevorzugtes erfindungsgemäß anzuwendendes oral konsumierbares Produkt (insbesondere Lebensmittel, Futtermittel und Arzneimittel) enthält nicht mehr als 200 kcal/100g des oral konsumierbaren Produktes, bevorzugt nicht mehr als 100 kcal/100g, besonders bevorzugt nicht mehr als 40 kcal/100g.

Bevorzugte erfindungsgemäß anzuwendende oral konsumierbare Produkte (insbesondere Lebensmittel, Futtermittel oder Arzneimittel) sind jegliche zum Verzehr geeignete der Ernährung, der Mundpflege oder dem Genuss dienende Zubereitungen oder Zusammensetzungen und sind regelmäßig Produkte, die dazu bestimmt sind, in die menschliche bzw. tierische Mundhöhle eingebracht zu werden, dort eine bestimmte Zeit zu verbleiben und anschließend entweder verzehrt (z.B. verzehrfertige Lebensmittel oder Futtermittel, siehe auch weiter unten) oder wieder aus der Mundhöhle entfernt zu werden (z.B. Kaugummis oder Mundpflegeprodukte oder medizinische Mundspülungen). Zu diesen Produkten gehören dabei sämtliche Stoffe oder Erzeugnisse, die dazu bestimmt sind, in verarbeitetem, teilweise verarbeitetem oder unverarbeitetem Zustand vom Menschen oder Tier aufgenommen zu werden. Hierzu zählen auch Stoffe, die oral konsumierbaren Produkten (insbesondere Lebensmittel, Futtermittel und Arzneimittel) bei ihrer Herstellung, Verarbeitung oder Bearbeitung zugesetzt werden und dazu vorgesehen sind, in die menschliche oder tierische Mundhöhle eingebracht zu werden.

Zu den erfindungsgemäß anzuwendenden oral konsumierbaren Produkten (insbesondere Lebensmittel, Futtermittel und Arzneimittel) zählen auch Stoffe, die dazu bestimmt sind, in unverändertem, zubereitetem oder verarbeitetem Zustand vom Menschen oder Tier geschluckt und dann verdaut zu werden; zu den erfindungsgemäß anzuwendenden oral konsumierbaren Produkten gehören insoweit auch Umhüllungen, Überzüge oder sonstige Umschließungen, die dazu bestimmt sind, mit verschluckt zu werden, oder bei denen ein Verschlucken vorauszusehen ist. Der Begriff "oral konsumierbares Produkt" umfasst verzehrfertige Lebensmittel und Futtermittel, d.h. Lebensmittel oder Futtermittel, die hinsichtlich der für den Geschmack maßgeblichen Substanzen bereits vollständig zusammengesetzt sind. Unter den Begriffen "verzehrfertiges Lebensmittel" oder "verzehrfertiges Futtermittel" fallen auch Getränke sowie feste oder halbfeste verzehrfertige Lebensmittel oder Futtermittel. Als Beispiele seien Tiefkühlprodukte genannt, die vor dem Verzehr aufgetaut und auf Verzehrtemperatur erwärmt werden müssen. Auch Produkte wie Joghurt oder Eiscreme aber auch Kaugummis oder Hartkaramellen zählen zu den verzehrfertigen Lebensmitteln oder Futtermitteln.

Bevorzugte erfindungsgemäß anzuwendende oral konsumierbare Produkte (insbesondere Lebensmittel und Futtermittel) umfassen auch "Halbfertigwaren". Unter einer Halbfertigware ist im Zusammenhang des vorliegenden Textes ein oral konsumierbares Produkt zu verstehen, welches aufgrund eines sehr hohen Gehaltes an Aroma- und Geschmacksstoffen für die Verwendung als verzehrfertiges oral konsumierbares Produkt (insbesondere Lebensmittel oder Futtermittel) ungeeignet ist. Erst durch Mischen mit mindestens einem weiteren Bestandteil (d.h. durch Verringern der Konzentration der betreffenden Aroma- und Ge-schmacksstoffe) und gegebenenfalls weitere Prozessschritte (z.B. Erwärmen, Gefrieren) wird die Halbfertigware in ein verzehrfertiges oral konsumierbares Produkt (insbesondere Lebensmittel oder Futtermittel) überführt. Als Beispiel für Halbfertigwaren seien hier Tütensuppen, Backaromen und Puddingpulver genannt.

Zu erfindungsgemäß anzuwendenden oral konsumierbaren Produkten (insbesondere Lebensmitteln, Futtermitteln oder Arzneimitteln) zählen auch "Mundpflegeprodukte". Unter einem Mundpflegeprodukt (auch Mundhygieneprodukt oder mundhygienische Zubereitung genannt) im Sinne der Erfindung wird eine der dem Fachmann geläufigen Formulierungen zur Reinigung und Pflege der Mundhöhle und des Rachenraumes sowie zur Erfrischung des Atems verstanden. Hierbei ist die Pflege der Zähne und des Zahnfleisches ausdrücklich eingeschlossen. Darreichungsformen gebräuchlicher mundhygienischer Formulierungen sind insbesondere Cremes, Gele, Pasten, Schäume, Emulsionen, Suspensionen, Aerosole, Sprays, wie auch Kapseln, Granulate, Pastillen, Tabletten, Bonbons oder Kaugummis, ohne dass diese Aufzählung für die Zwecke dieser Erfindung limitierend verstanden werden soll.

Bevorzugt umfasst ein erfindungsgemäß anzuwendendes oral konsumierbares Produkt (insbesondere Lebensmittel oder Futtermittel), eine oder mehrere der Ernährung oder dem Genuss dienende Zubereitungen. Hierunter fallen insbesondere (kalorienreduzierte) Backwaren (z.B. Brot, Trockenkekse, Kuchen, sonstiges Gebäck), Süßwaren (z.B. Schokoladen, Schokoladenriegelprodukte, sonstige Riegelprodukte, Fruchtgummi, Dragees, Hart- und Weichkaramellen, Kaugummi), nicht-alkoholische Getränke (z.B. Kakao, Kaffee, Grüntee, Schwarztee, mit (Grün-, Schwarz-)Tee-Extrakten angereicherte (Grün-, Schwarz-)Tee-Getränke, Rooibos-Tee, andere Kräutertees, fruchthaltige Limonaden, isotonische Getränke, Erfrischungsgetränke, Nektare, Obst- und Gemüsesäfte, Frucht- oder Gemüsesaftzubereitungen), Instantgetränke (z.B. Instant-Kakao-Getränke, Instant-Tee-Getränke, Instant-Kaffeegetränke), Fleischprodukte (z.B. Schinken, Frischwurst- oder Rohwurstzubereitungen, gewürzte oder marinierte Frisch- oder Pökelfleischprodukte), Insekten oder Insektenprodukten, Eier oder Eiprodukte (Trockenei, Eiweiß, Eigelb), Getreideprodukte (z.B. Frühstückscerealien, Müsliriegel, vorgegarte Fertigreis-Produkte), Milchprodukte (z.B. Vollfett- oder fettreduzierte oder fettfreie, bevorzugt aber proteinreiche Milchgetränke, Milchreis, Joghurt, Kefir, Frischkäse, Weichkäse, Hartkäse, Trockenmilchpulver, Molke, Butter, Buttermilch, teilweise oder ganz hydrolisierte Milchprotein-haltige Produkte), Produkte mit hohem Anteil veganer, bevorzugt pflanzlicher Proteine, z.B. Algen-, Weizen-, Raps-, Sonnenblumen-, Reis-, Kartoffel-, Mais-, Soja- , Bohnen-, Erbsen-, Linsen-, Lupinen-, Erdnuss-, Alfalfa-Proteinisolaten, Proteinfraktionen, partiellen oder vollständigen Proteinhydrolysaten, oder physikalisch-chemisch, enzymatisch oder fermentativ veränderten Proteinen der vorgenannten Quellen, so z.B. aus Sojaprotein oder anderen Sojabohnen-Fraktionen (z.B. Sojamilch und daraus gefertigte Produkte, Getränke, enthaltend isoliertes oder enzymatisch behandeltes Sojaprotein, Sojamehl enthaltende Getränke, Sojalecithin-haltige Zubereitungen, fermentierte Produkte wie Tofu oder Tempe oder daraus gefertigte Produkte und Mischungen mit Fruchtzubereitungen und fakultativ Aromen), Fleischersatzprodukte aus vorgenannten vegetarischen oder veganen Proteinquellen, Fruchtzubereitungen (z.B. Konfitüren, Fruchteis, Fruchtsoßen, Fruchtfüllungen), Gemüsezubereitungen (z.B. Ketchup, Soßen, Trockengemüse, Tiefkühlgemüse, vorgegartes Gemüse, eingekochtes Gemüse), Knabberartikel (z.B. gebackene oder frittierte Kartoffel-chips oder Kartoffelteigprodukte, Extrudate auf Mais- oder Erdnussbasis), Produkte auf Fett- und Ölbasis oder Emulsionen derselben (z.B. Mayonnaise, Remoulade, Dressings, jeweils Vollfett- oder fettreduziert), sonstige Fertiggerichte und Suppen (z.B. Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden, Süßstoffzubereitungen, -tabletten oder -sachets, sonstige Zubereitungen zum Süßen oder Weißen von Getränken oder anderen Nahrungsmitteln. Die Zubereitungen im Sinne der Erfindung können auch als Halbfertigware zur Herstellung weiterer der Ernährung oder dem Genuss dienenden Zubereitungen dienen. Die Zubereitungen im Sinne der Erfindung können auch in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen und als Nahrungsergänzungsmittel vorliegen.

Besonders bevorzugt sind kalorienreduzierte Süßwaren (z.B. Müsliriegelprodukte, Fruchtgummi, Dragees, Hart- und Weichkaramellen, Kaugummi), nicht-alkoholische Getränke (z.B. Kakao, Grüntee, Schwarztee, mit (Grün-, Schwarz-)Tee-Extrakten ange-reicherte (Grün-, Schwarz-)Tee-Getränke, Rooibos-Tee, andere Kräutertees, fruchthaltige Limonaden, isotonische Getränke, Erfrischungsgetränke, Nektare, Obst- und Gemüsesäfte, Frucht- oder Gemüsesaftzubereitungen), Instantgetränke (z.B. Instant-Kakao-Getränke, Instant-Tee-Getränke), Getreideprodukte (z.B. Frühstückscerealien, Müsliriegel, vorgegarte Fertigreis-Produkte), Milchprodukte (z.B. Vollfett- oder fettreduzierte oder fettfreie Milchgetränke, Milchreis, Joghurt, Kefir, Trockenmilchpulver, Molke, Buttermilch, teilweise oder ganz hydrolisierte Milchprotein-haltige Produkte), Produkte mit hohem Anteil veganer, bevorzugt pflanzlicher Proteine, z.B. Algen-, Weizen-, Raps-, Sonnenblumen-, Reis-, Kartoffel-, Mais-, Soja- , Bohnen-, Erbsen-, Linsen-, Lupinen-, Erdnuss-, Alfalfa-Proteinisolaten, Proteinfraktionen, partiellen oder vollständigen Proteinhydrolysaten, oder physikalisch-chemisch, enzymatisch oder fermentativ veränderten Proteinen der vorgenannten Quellen, Produkte aus Sojaprotein oder anderen Sojabohnen-Fraktionen (z.B. Sojamilch und daraus gefertigte Produkte, Getränke, enthaltend isoliertes oder enzymatisch behandeltes Sojaprotein, Sojamehl enthaltende Getränke, Sojalecithin-haltige Zubereitungen, fermentierte Produkte wie Tofu oder Tempe oder daraus gefertigte Produkte und Mischungen mit Fruchtzubereitungen und fakultativ Aromen), Süßstoffzubereitungen, -tabletten oder -sachets, sonstige Zubereitungen zum Süßen oder Weißen von Getränken oder anderen Nahrungsmitteln, Gemüsezubereitungen (z.B. Ketchup, Soßen, Trockengemüse, Tiefkühlgemüse, vorgegartes Gemüse, eingekochtes Gemüse), Fertiggerichte und Suppen (z.B. Trockensuppen, Instant-Suppen, vorgegarte Suppen) Fleischersatzprodukte aus vorgenannten vegetarischen oder veganen Proteinquellen.

Die Zubereitungen, insbesondere oral konsumierbare Produkte, können auch in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen vorliegen, z. B. als Nahrungsergänzungsmittel.

Die Halbfertigwaren dienen in der Regel zur Herstellung von der Ernährung oder dem Genuss dienenden gebrauchs- oder verzehrfertigen Zubereitungen.

Weitere Bestandteile einer der Ernährung oder dem Genuss dienenden verzehrfertigen Zubereitung bzw. Halbfertigware können übliche Grund-, Hilfs- und Zusatzstoffe für Nahrungs- oder Genussmittel sein, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Kräuter, Nüsse, Gemüsesäfte oder - pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose, Tagatose), Zuckeralkohole (z.B. Sorbit, Erythritol), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nicht-proteinogene Aminosäuren und verwandte Verbindungen (z.B. γ-Aminobuttersäure, Taurin), Peptide (z.B. Glutathion), native oder prozessierte Proteine (z.B. Gelatine), Enzyme (z.B. Peptidasen), Nucleinsäuren, Nucleotide, Geschmackskorrigenzien für unangenehme Geschmackseindrücke, weitere Geschmacksmodulatoren für weitere, in der Regel nicht unangenehme Geschmackseindrücke, andere geschmacks-modulierende Stoffe (z.B. Inositolphosphat, Nucleotide wie Guanosinmonophosphat, Adenosin-monophosphat oder andere Stoffe wie Natriumglutamat oder 2-Phen-oxypropionsäure), Emulgatoren (z.B. Lecithine, Diacylglycerole, Gummi arabicum), Stabilisatoren (z.B. Carrageenan, Alginat), Konservierungsstoffe (z.B. Benzoesäure und deren Salze, Sorbinsäure und deren Salze), Antioxidantien (z.B. Tocopherol, Ascorbin-säure), Chelatoren (z.B. Citronensäure), organische oder anorganische Säuerungsmittel (z.B. Essigsäure, Phosphorsäure), zusätzliche Bitterstoffe (z.B. Chinin, Coffein, Limonin, Amarogentin, Humolone, Lupolone, Catechine, Tannine), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, trigeminal wirksame Stoffe oder Pflanzenextrakte, enthaltend solche trigeminal wirksamen Stoffe, synthetische, natürliche oder naturidentische Aromastoffe oder Riechstoffe sowie Geruchskorrigenzien.

Bevorzugt enthalten erfindungsgemäße oral konsumierbare Produkte (insbesondere Lebensmittel, Futtermittel und Arzneimittel), z.B. solche in Form von Zubereitungen oder Halbfertigwaren, eine Aromakomposition, um den Geschmack und/oder den Geruch abzurunden und zu verfeinern. Eine Zubereitung kann als Bestandteile einen festen Trägerstoff und eine Aromakomposition umfassen. Geeignete Aromakompositionen enthalten z.B. synthetische, natürliche oder naturidentische Aroma-, Riech- und Ge-schmacksstoffe, Reaktionsaromen, Raucharomen oder andere aromagebende Zuberei-tungen (z.B. Protein[teil]hydrolysate, bevorzugt Protein[teil]hydrolysate mit hohem Arginin-Gehalt, Grillaromen, Pflanzenextrakte, Gewürze, Gewürzzubereitungen, Gemüse und/oder Gemüsezubereitungen) sowie geeignete Hilfs- und Trägerstoffe. Insbesondere sind hier die Aromenkompositionen oder deren Bestandteile geeignet, die einen röstigen, fleischigen (insbesondere Huhn, Fisch, Meerestiere, Rind, Schwein, Lamm, Schaf, Ziege), gemüsigen (insbesondere Tomate, Zwiebel, Knoblauch, Sellerie, Lauch, Pilze, Auberginen, Seetang), einen würzigen (insbesondere schwarzer und weißer Pfeffer, Kardamom, Muskat, Piment, Senf und Senf-Produkte), gebratenen, hefigen, gesotteen, fettigen, salzigen und/oder scharfen Aromaeindruck verursachen und somit den würzigen Eindruck verstärken können. In der Regel enthalten die Aromakompositionen mehr als einen der genannten Inhaltsstoffe.

Die Energiedichte eines oral konsumierbaren Produktes (insbesondere Lebensmittels, Futtermittels oder Arzneimittels) lässt sich senken, indem energiereiche Inhaltsstoffe des oral konsumierbaren Produktes gegen Ersatzstoffe (z.B. kalorienarme Verdickungsmittel statt Fette, kalorienarme oder kalorienfreie Süßstoffe statt üblicher Zucker) ausgetauscht werden. Der bereits oben diskutierte Nachteil, dass der Konsument ein oral konsumierbares Produkt (insbesondere ein Lebensmittel) mit reduzierter Energiedichte in größeren Mengen konsumiert und es dann im ungünstigsten Fall sogar zu einer erhöhten Aufnahme kalorisch relevanter Nahrungsmittelbestandteile kommt, wird durch die Verwendung des aromatischen Alkensäurederivats oder eines Gemischs von aromatischen Alkensäurederivate in oral konsumierbaren Lebensmitteln (insbesondere in Lebensmitteln) entgegengewirkt. In einem oral konsumierbaren Produkt enthaltenes aromatisches Alkensäu-rederivat oder eines Gemischs von aromatischen Alkensäurederivaten vermittelt ein vorzeitiges Gefühl von Sättigung und reduziert gleichzeitig den Appetit und die Resorption von Lipiden. Zudem hat es einen positiven Einfluss auf die Stimmung des Konsumenten, was sich ebenfalls positiv auswirkt.

Vorzugsweise ist ein erfindungsgemäß anzuwendendes oral konsumierbares Produkt ausgewählt aus der Gruppe bestehend aus Süßwaren, vorzugsweise kalorienreduzierte bzw. kalorienfreie Süßwaren, vorzugsweise ausgewählt aus der Gruppe bestehend aus Müsliriegelprodukte, Fruchtgummi, Dragees, Hartkramellen und Kaugummi,
- nicht-alkoholische Getränke, vorzugsweise ausgewählt aus der Gruppe bestehend aus Grüntee, Schwarztee, mit (Grün-, Schwarz-)Tee-Extrakten angereicherte (Grün-, Schwarz-)Tee-Getränke, Rooibos-Tee, andere Kräutertees, fruchthaltige zuckerarme oder -freie Limonaden, isotonische Getränke, Nektare, Obst- und Gemüsesäfte, Frucht- und Gemüsesaftzubereitungen,
- Instantgetränke, vorzugsweise ausgewählt aus der Gruppe bestehend aus Instant- (Grün-, Schwarz-, Rooibos-, Käuter-)Tee-Getränke,
- Getreideprodukte, vorzugsweise ausgewählt aus der Gruppe bestehend aus zuckerarmen und -freien Frühstückscerealien und Müsliriegeln,
- Milchprodukte, vorzugsweise ausgewählt aus der Gruppe bestehend aus fettreduzierten und fettfreien Milchgetränken, Joghurt, Kefir, Molke, Buttermilch und Eiscreme,
- Produkte aus Sojaprotein oder anderen Sojabohnen-Fraktionen, vorzugsweise ausgewählt aus der Gruppe bestehend aus Sojamilch, aus Sojamilch gefertigten Produkten, Getränken enthaltend isoliertes oder enzymatisch behandeltes Sojaprotein, Sojamehl enthaltenden Getränken, Sojalecithin-haltigen Zubereitungen, aus Sojalecithin-haltigen Zubereitungen gefertigten Produkten und Mischungen mit Fruchtzubereitungen und ggf. Aromen,
- Produkte aus anderen pflanzlichen Proteinen bzw. Proteinfraktionen, wobei die Quelle für die Proteine bevorzugt ausgewählt aus der Gruppe bestehend aus Süßlupine, Erbse, Bohne, Linse, Weizen, Raps, Algen, Kartoffeln und Reis,
- Süßstoffzubereitungen, -tabletten und -sachets
- Zuckerfreie Dragees,
- Eiscreme, mit oder ohne Bestandteile auf Basis von Milch, vorzugsweise zuckerfrei.
- Herzhafte ("Savory") Lebensmittel, vorzugsweise ausgewählt aus der Gruppe bestehend aus Fleischersatzprodukten, Nudelgerichten, Suppen, proteinreichen Suppen, etc..

Bevorzugt enthält ein erfindungsgemäß anzuwendendes oral konsumierbares Produkt (insbesondere Lebensmittel, Futtermittel oder Arzneimittel) (a) einen, zwei oder mehrere Süßstoffe und/oder (b) ein, zwei oder mehrere Verdickungsmittel.

Der Begriff "Süßstoffe" bezeichnet hierbei Stoffe mit einer relativen Süßkraft von zumindest 25, bezogen auf die Süßkraft von Saccharose (welches somit die Süßkraft 1 hat). Vorzugsweise sind in einem erfindungsgemäß anzuwendenden oral konsumierbaren Produkt (insbesondere Lebensmittel, Futtermittel oder Arzneimittel) (a) einzusetzende Süßstoffe nicht-kariogen und/oder besitzen einen Energiegehalt von maximal 5 kcal pro Gramm des oral konsumierbaren Produktes.

Vorteilhafte Süßstoffe in einem bevorzugten erfindungsgemäß anzuwendenden oral konsumierbaren Produkt (insbesondere Lebensmittel, Futtermittel oder Arzneimittel) sind ausgewählt aus den folgenden Gruppen (a1) und (a2):
(a1) natürlich vorkommende Süßstoffe, vorzugsweise ausgewählt aus der Gruppe bestehend aus
(a1-1) Miraculin, Monellin, Mabinlin, Thaumatin, Curculin, Brazzein, Pentaidin, D-Phenylalanin, D-Tryptophan, und aus natürlichen Quellen gewonnene Extrakte oder Fraktionen, enthaltend diese Aminosäuren und/oder Proteine, und den physio-logisch akzeptablen Salzen dieser Aminosäuren und/oder Proteine, insbesondere den Natrium-, Kalium-, Calcium oder Ammoniumsalzen;
(a1-2) Neohesperidindihydrochalkon, Naringindihydrochalkon, Steviosid, Steviolbiosid, Rebaudiosiden, insbesondere Rebaudiosid A, Rebaudiosid B, Rebaudiosid C, Rebaudiosid D, Rebaudiosid E, Rebaudiosid F, Rebaudiosid G, Rebaudiosid H, Rebaudiosid M, Rebaudiosid X, Dulcosiden und Rubusosid, Suavioside A, Suavioside B, Suavioside G, Suavioside H, Suavioside I, Suavioside J, Baiyunoside 1 Baiyunoside 2, Phlomisoside 1, Phlomisoside 2, Phlomisoside 3, sowie Phlomisoside 4, Abrusoside A, Abrusoside B, Abrusoside C, Abrusoside D, Cyclocaryoside A und Cyclocaryoside I, Oslandin, Polypodosid A, Strogin 1, Strogin 2, Strogin 4, Selligueanin A, Dihydroquercetin-3-acetat, Perillartin, Telosmosid A15, Periandrin I-V, Pterocaryosiden, Cyclocaryosiden, Mukuroziosiden, trans-Anethol, trans-Cinnamaldehyd, Bryosiden, Bryonosiden, Bryonodulcosiden, Carnosiflosiden, Scandenosiden, Gypenosiden, Trilobatin, Phloridzin, Dihydroflavanolen, Hematoxylin, Cyanin, Chlorogensäure, Albiziasaponin, Telosmosiden, Gaudichaudiosid, Mogrosiden, Mogrosid V, Hernandulcinen, Monatin, Phyllodulcin, Glycyrrhetinsäure und deren Derivaten, insbesondere deren Glycosiden wie Glycyrrhizin, und den physiologisch akzeptablen Salzen dieser Verbindungen, insbesondere den Natrium-, Kalium-, Calcium oder Ammoniumsalzen;
(a1-3) Extrakte oder angereicherte Fraktionen der Extrakte, ausgewählt aus der Gruppe bestehend aus Thaumatococcus-Extrakten (Katemfestaude), Extrakten aus Stevia ssp. (insbesondere Stevia rebaudiana), Swingle-Extrakten (Momordica bzw. Siratia grosvenorii, Luo-Han-Guo), Extrakten aus Glycerrhyzia ssp. (insbesondere Gly-cerrhyzia glabra), Extrakten aus Rubus ssp. (insbesondere Rubus suavissimus), Citrus-Extrakten und Extrakten aus Lippia dulcis;
(a2) synthetische süß schmeckende Stoffe, vorzugsweise ausgewählt aus der Gruppe bestehend aus Magap, Natriumcyclamat oder anderen physiologisch akzeptablen Salzen der Cyclamsäure, Acesulfam K oder anderen physiologisch akzeptablen Salzen des Acesulfam, Neohesperidindihydrochalkon, Naringindihydrochalkon, Saccharin, Saccharin-Natriumsalz, Aspartam, Superaspartam, Neotam, Alitam, Advantam, Perillartin, Sucralose, Lugduname, Carrelame, Sucrononate und Sucrooctate.

Vorteilhafte Verdickungsmittel in einem bevorzugten erfindungsgemäß anzuwendenden oral konsumierbaren Produkt (insbesondere Lebensmittel, Futtermittel oder Arzneimittel) sind ausgewählt aus der Gruppe bestehend aus: vernetzte Polyacrylsäuren und deren Derivate, Polysaccharide und deren Derivate, wie Xanthangummi, Agar-Agar, Alginate oder Tylosen, Cellulosederivate, z. B. Carboxymethylcellulose oder Hydroxycarboxymethylcellulose, Fettalkohole, Monoglyceride und Fettsäuren, Polyvinylalkohol und Polyvinylpyrrolidon.

Erfindungsgemäß bevorzugt ist ein oral konsumierbares Produkt (insbesondere Lebensmittel oder Futtermittel), das durch Milchsäurebakterien verdickte Milch und/oder durch Milchsäurebakterien verdickte Sahne enthält und vorzugsweise ausgewählt ist aus der Gruppe bestehend aus oral konsumierbaren Produkten mit einem Fettgehalt von 4,0 Gew.-% oder weniger, bevorzugt von 1,5 Gew.-% oder weniger, besonders bevorzugt 0,5 Gew.-% oder weniger, jeweils bezogen auf das Gesamtgewicht des oral konsumierbaren Produktes, und/oder ausgewählt ist aus der Gruppe bestehend aus Joghurt, Kefir und Quark.

Vorzugsweise enthält das erfindungsgemäß anzuwendende oral konsumierbare Produkt (insbesondere Lebensmittel oder Futtermittel), das durch Milchsäurebakterien verdickte Milch und/oder durch Milchsäurebakterien verdickte Sahne enthält, einen Energiegehalt von nicht mehr als 150 kcal/100g des oral konsumierbaren Produktes, bevorzugt nicht mehr als 100 kcal/100g, besonders bevorzugt nicht mehr als 75 kcal/100g, besonders bevorzugt nicht mehr als 50 kcal/100g.

Ein bevorzugtes erfindungsgemäß anzuwendendes oral konsumierbares Produkt (insbesondere Lebensmittel oder Futtermittel), das durch Milchsäurebakterien verdickte Milch und/oder durch Milchsäurebakterien verdickte Sahne enthält, umfasst zusätzlich Früchte und/oder Fruchtzubereitungen.

Besonders bevorzugt ist ein erfindungsgemäß anzuwendendes oral konsumierbares Produkt (insbesondere Lebensmittel oder Futtermittel), das durch Milchsäurebakterien verdickte Milch und/oder durch Milchsäurebakterien verdickte Sahne enthält, wobei das oral konsumier-bare Produkt (i) Zucker und/oder (ii) Verdickungsmittel und/oder (iii) Geliermittel und/oder (iv) Süßungsmittel und/oder (v) Aromen und/oder (vi) Konservierungsstoffe enthält.

"Zucker" ist im Rahmen des vorliegenden Textes (sofern nicht anders angegeben oder aus dem Kontext anders ersichtlich) der Sammelbegriff für alle süß schmeckenden Saccharide (Einfach- und Doppelzucker).

Vorteilhafterweise ist ein erfindungsgemäß anzuwendendes oral konsumierbares Produkt (insbesondere Lebensmittel oder Futtermittel), das durch Milchsäurebakterien verdickte Milch und/oder durch Milchsäurebakterien verdickte Sahne enthält, ein oral konsumierbares Produkt, das ein Probiotikum enthält, wobei das Probiotikum vorzugsweise ausgewählt ist aus der Gruppe bestehend aus Bifidobacterium animalis subsp. lactis BB-12, Bifidobacterium animalis subsp. lactis DN-173 010, Bifidobacterium animalis subsp. lactis HN019, Lacto-bacillus acidophilus LA5, Lactobacillus acidophilus NCFM, Lactobacillus johnsonii La1, Lactobacillus casei immunitass/defensis, Lactobacillus casei Shirota (DSM 20312), Lactobacillus casei CRL431, Lactobacillus reuteri (ATCC 55730) und Lactobacillus rham-nosus (ATCC 53013).

Besonders bevorzugt ist ein erfindungsgemäß anzuwendendes oral konsumierbares Produkt (insbesondere Lebensmittel, Futtermittel oder Arzneimittel), das ein Kaugummi ist und eine Kaugummibase enthält. Die Kaugummibase ist dabei vorzugsweise ausgewählt aus der Gruppe bestehend aus "chewing gum" - oder "bubble gum" - Basen. Letztere sind weicher, damit sich damit auch Kaugummiblasen bilden lassen. Erfindungsgemäß bevorzugte Kaugummibasen umfassen neben traditionell eingesetzten natürlichen Harzen oder dem Naturlatex Chicle Elastomere wie Polyvinylacetate (PVA), Polyethylene, (nieder- oder mittelmolekulare) Polyisobutene (PIB), Polybutadiene, Isobuten-Isopren Copolymere (Butyl Rubber), Polyvinylethylether (PVE), Polyvinylbutylether, Copolymere von Vinylestern und Vinylethern, Styrol-Butadien-Copolymere (Styrol-Butadien-Rubber, SBR) oder Vinyl-Elastomere, z.B. auf Basis Vinylacetat/Vinyllaurat, Vinylacetat/Vinylstaerat oder Ethylen/Vinylacetat, sowie Mischungen der genannten Elastomere, wie beispiels-weise in EP 0 242 325, US 4,518,615, US 5,093,136, US 5,266,336, US 5,601,858 oder US 6,986,709 beschrieben. Daneben umfassen erfindungsgemäß bevorzugt einzusetzende Kaugummibasen vorzugsweise weitere Bestandteile wie beispielsweise (mineralische) Füllstoffe, Weichmacher, Emulgatoren, Antioxidantien, Wachse, Fette oder fette Öle, wie beispielsweise gehärtete (hydrierte) pflanzliche oder tierische Fette, Mono-, Di- oder Triglyceride. Geeignete (mineralische) Füllstoffe sind beispielsweise Calciumcarbonat, Titandioxid, Siliciumdioxid, Talkum, Aluminiumoxid, Dicalciumphosphat, Tricalciumphosphat, Magnesiumhydroxid und deren Mischungen. Geeignete Weichmacher bzw. Mittel zur Verhinderung des Verklebens (detackifier) sind beispielsweise Lanolin, Stearin-säure, Natriumstearat, Ethylacetat, Diacetin (Gylcerindiacetat), Triacetin (Gylcerintriacetat), Triethylcitrat. Geeignete Wachse sind beispielsweise Paraffin Wachse, Candelilla Wachs, Carnauba Wachs, mikrokristalline Wachse und Polyethylen Wachse. Geeignete Emulgatoren sind beispielsweise Phosphatide wie Lecithin, Mono- und Diglyceride von Fettsäuren, z.B. Glycerinmonostearat.

Erfindungsgemäß anzuwendende Kaugummis (insbesondere wie vorstehend offenbart) umfassen vorzugsweise Bestandteile wie Zucker verschiedener Arten, Zuckeraustauschstoffe, andere süß schmeckende Stoffe, Zuckeralkohole (insbesondere Sorbitol, Xylitol, Mannitol), Kühlwirkstoffe, Geschmackskorrigenzien für unangenehme Geschmackseindrücke, weitere geschmacksmodulierende Stoffe (z.B. Inositolphosphat, Nucleotide wie Guano-sinmonophosphat, Adenosinmonophosphat oder andere Stoffe wie Natriumglutamat oder 2-Phenoxypropionsäure), Feuchthaltemittel, Verdicker, Emulgatoren, Stabilisatoren, Geruchskorrigentien und Aromen (z.B.: Eycalyptus-Menthol, Kirsche, Erdbeere, Grapefruit, Vanille, Banane, Citrus, Pfirsich, schwarze Johannisbeere, Tropenfrüchte, Ingwer, Kaffee, Zimt, Kombinationen (der genannten Aromen) mit Mintaromen sowie Spearmint und Pfefferminz alleine). Besonders interessant ist unter anderem die Kombination der Aromen mit weiteren Stoffen, die kühlende, wärmende und/oder mundwässernde Eigenschaften aufweisen.

Besonders bevorzugt ist ein erfindungsgemäß anzuwendendes oral konsumierbares Produkt (insbesondere Lebensmittel, Futtermittel oder Arzneimittel), wobei das oral konsumierbare Produkt ein Getränk ist, wobei das Getränk vorzugsweise einen Zuckergehalt von 30 g/100 mL Getränk oder weniger, bevorzugt von 15 g/100 mL oder weniger, besonders bevorzugt 5 g/100 mL oder weniger aufweist, besonders bevorzugt kein Zucker enthält
und/oder
wobei das Getränk kein Ethanol oder maximal 0,1 Volumenprozent Ethanol enthält, bezogen auf das Volumen des Getränkes.

Im Rahmen der vorliegenden Erfindung sind erfindungsgemäß anzuwendende oral konsumierbare Produkte weniger bevorzugt, bei denen es sich um Ethanol enthaltende Getränke handelt.

Kein Ethanol bedeutet im Zusammenhang mit der vorliegenden Erfindung, dass kein Ethanol zugesetzt wird und dass die Zubereitung weniger als 0,1 Vol.-%, bevorzugt weniger als 0,01 Vol.-% und besonders bevorzugt keine messbare Menge an Ethanol enthält.

Besonders bevorzugt sind erfindungsgemäß anzuwendende oral konsumierbare Produkte (vorzugsweise Lebensmittel, Futtermittel oder Arzneimittel), wobei es sich um ein kohlensäurehaltiges Getränk oder um ein kohlensäurefreies Getränk handelt.

Gemäß einem weiteren Aspekt betrifft die vorliegende Erfindung die nicht-therapeutische Verwendung eines oder einer Mischung von zwei oder mehr Cinnamylalkoholderivats/en nach Formel I 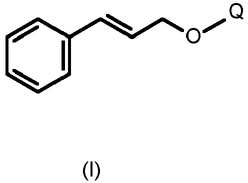
wobei die Doppelbindung eine E- oder Z-Konfiguration aufweisen kann oder eine beliebige Mischung von E- und Z-Konfigurationen vorliegen kann, und wobei Q ein Acylrest der Formel II 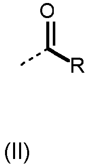
ist, wobei R Wasserstoff oder ein gesättigter oder ungesättigter, linearer oder verzweigter oder cylischer aliphatischer Rest mit 2 bis 6 Kohlenstoffatomen oder Phenyl oder Phenylmethyl ist als (a) Mittel zur Reduzierung des Appetits und/oder (b) Mittel zur Vermittlung eines Gefühls von Sättigung und/oder als (c) Mittel zur Reduzierung der Energieaufnahme und/oder (d) Mittel zur Reduzierung des Körpergewichts und/oder als (e) Stimmungsaufheller oder
Cinnamylalkoholderivat der Formel I, wobei Q ein Acylrest der Formel II und R Methyl ist, als (a) Mittel zur Reduzierung des Appetits und/oder (b) Mittel zur Vermittlung eines Gefühls von Sättigung und/oder als (c) Mittel zur Reduzierung der Energieaufnahme und/oder (d) Mittel zur Reduzierung des Körpergewichts.

Bevorzugt einzusetzende Cinnamylalkoholderivate der Formel I, sowie deren Mischungen entsprechen den oben beschriebenen Ausführungsformen. Das im Zusammenhang mit den oben beschriebenen Ausführungsformen Gesagte gilt entsprechend.

Gemäß noch einem weiteren bevorzugten Aspekt betrifft die vorliegende Erfindung ferner ein nicht-therapeutisches Verfahren zur (i) Reduzierung des Appetits und/oder (ii) Vermittlung eines Gefühls von Sättigung und/oder (iii) Reduzierung der Energieaufnahme und/oder (iv) Reduzierung des Körpergewichts und/oder (v) Aufhellung der Stimmung umfassend den Schritt
- Verabreichen einer (a) den Appetit reduzierenden und/oder (b) ein Gefühl von Sättigung bewirkenden und/oder (c) einer stimmungsaufhellenden Menge von einem oder einer Mischung aus zwei oder mehr Cinnamylalkoholderivaten der Formel I wie oben beschrieben oder von einer Zubereitung wie oben beschrieben an ein Individuum.

Gemäß dem nicht-therapeutischen Verfahren, werden die erfindungsgemäß anzuwendenden Cinnamylalkoholderivate der Formel I vorzugsweise zur Reduzierung der kalorischen Aufnahme und dabei bevorzugt zur kosmetischen Reduzierung des Körpergewichts eingesetzt.

Bevorzugt zu verabreichende Cinnamylalkoholderivate der Formel I, sowie deren Mischungen entsprechen den oben beschriebenen Ausführungsformen. Das im Zusammenhang mit den oben beschriebenen Ausführungsformen Gesagte gilt entsprechend.

Eine nicht-therapeutische Verwendung oder ein nicht-therapeutisches Verfahren im Sinne der vorliegenden Erfindung betrifft Verwendungen und Verfahren, die nicht zur chirurgischen oder therapeutischen Behandlung eines pathologischen Symptoms oder Krankheitsbildes gedacht sind. Vorzugsweise bezieht sich der Begriff "nicht-therapeutisch" auf die Erhaltung von normalen bzw. gesunden Zuständen durch Nahrungs- oder Nahrungsergänzungsmittel, z.B. Verfahren zur Erhaltung des normalen oder Senkung des nicht-krankhaften Körpergewichts und/oder des Sättigungsgefühls, sowie kosmetische Verfahren oder kosmetische Anwendungen.

Die Erfindung betrifft auch ein nicht-therapeutisches Verfahren wie oben beschrieben, wobei das bzw. die Cinnamylalkoholderivat(e) der Formel I in Form einer Zubereitung wie oben beschrieben, insbesondere in Form eines oral konsumierbaren Produkts wie oben beschrieben, verabreicht werden.

Bevorzugt einzusetzende Zubereitungen, insbesondere oral konsumierbare Produkte, entsprechen den oben beschriebenen Ausführungsformen. Das im Zusammenhang mit den oben beschriebenen Ausführungsformen Gesagte gilt entsprechend.

Weitere Aspekte der vorliegenden Erfindung ergeben sich aus den nachfolgenden Beispielen und den beigefügten Ansprüchen.

### Beispiel 1: Experimentelles Zellsystem

Als Zellmodell für die Ausschüttung des Neurotransmitters Serotonin werden humane Neuroblastomazellen (SH-SY5Y, ATCC Nummer CRL-2266) verwendet. Die Kultivierung erfolgt bei 37 °C und 5 % CO2-Gehalt mit einer Mischung, die zu gleichen Teilen aus Eagle's Minimum Essential Medium (EMEM) und F12 Medium (jeweils mit 10 % FBS und 1 % Penicillin/Streptomycin) besteht. Zur Bestimmung der Serotoninfreisetzung werden die Zellen mit Trypsin geerntet und nach einer Vitalitätsprüfung durch Trypanblaufärbung in definierter Zellzahl in 24-well Platten (Corning cell culture treated) ausgesät.

### Beispiel 2: Freisetzung von Serotonin in einem experimentellen Zellsystem mit Verbindungen 1 bis 4

Nach 5-minütiger Stimulierung von 90.000 humanen Neuroblastomazellen (SH SY5Y) mit 60 µl Krebs-Ringer-HEPES Puffer, 0,1 % Ascorbinsäure, pH 6,2, mit oder ohne Zusatz von 10 µM einer der Verbindungen 1-4, , bzw. 10 µM Nonivamid als Positivkontrolle wird der Serotoningehalt mit Hilfe eines enzymbasierten Nachweisverfahrens (Serotonin-ELISA sensitiv, DLD Diagnostica, Hamburg, Deutschland) ermittelt. Tabelle 1. Serotonin-Freisetzung in SH-SY5Y-Zellen nach Stimulierung mit je 10 µM von Verbindungen nach Formel I.

| **Testsubstanz** | **Serotoninfreisetzung [% der Positivkontrolle]** | **Standardabweichung [%]** |
|---|---|---|
| 10 µM Nonivamid (Positivkontrolle) | 100 | 2.60 |
| Verbindung 1 (Cinnamylalkohol, nicht erfindungsgemäß) | 129.29 | 2.02 |
| Verbindung 4 | 115.57 | 5.94 |
| Verbindung 6 | 120.69 | 5.26 |
| Verbindung 7 | 118.47 | 2.06 |
| Verbindung 11 | 120.83 | 0.73 |

Tabelle 1 zeigt den Einfluss der Verbindungen 1, 4, 6, 7 und 11 auf die Serotoninfreisetzung in SH-SY5Y Zellen, bezogen auf die Stimulation mit 10 µM Nonivamid (Positivkontrolle). Dabei zeigt Verbindung 1 eine ca. 30%ige Steigerung der Serotoninfreisetzung aus SH-SY5Y-Zellen gegenüber der Positivkontrolle, während die übrigen Verbindungen eine Steigerung der Serotoninfreisetzung um ca. 15-20% gegenüber der Positivkontrolle bewirken.

### Anwendungsbeispiel 1 : Erfrischungsgetränke (zuckerhaltig, kalorienreduziert, kalorienfrei)

Die Zutaten wurden in der angegebenen Reihenfolge gemischt und mit Wasser auf 100 % aufgefüllt. Die Mischungen werden in Glasflaschen gefüllt und karbonisiert.

| Inhaltsstoff | Einsatz in Gew.-% | | | | | | |
|---|---|---|---|---|---|---|---|
| Zubereitung | A (nicht erfindungsgemäß) | B | C | D (nicht erfindungsgemäß) | E (nicht erfindungsgemäß) | F (nicht erfindungs gemäß) | G |
| Zucker (Sucrose) | 10 | 10 | 7 | - | - | 8 | 7 |
| Glucose/Fructose-Sirup aus Mais, enthaltend 55 Gew.-% Fructose | - | - | - | - | 10 | - | - |
| Rebaudiosid A 95 % | - | - | 0,02 | 0,05 | - | - | - |
| Zitronensäure | 0,15 | 0,15 | 0,06 | 0,15 | 0,15 | 0,15 | 0,15 |
| Phosphorsäure | - | - | 0,07 | - | - | - | - |
| Zuckercouleur | - | - | 0,14 | - | - | - | - |
| Coffein | - | - | 0,01 | - | - | - | - |
| Zitronenaroma | 0,1 | 0,05 | - | 0,1 | 0,1 | 0,1 | 0,1 |
| Limonenaroma | - | 0,05 | - | - | - | - | - |
| Getränke-Emulsion Typ "Cola" | - | - | 0,05 | - | - | - | - |
| Phloretin | - | - | 0,002 | 0,003 | - | 0,002 | 0,001 |
| Hesperetin | - | - | 0,001 | 0,002 | - | - | 0,002 |
| Extrakt aus Rubus suavissimus, enthaltend 5 Gew.-% Rubusosid bez. auf das Gesamtgewicht des Extraktes | - | - | - | - | - | 0,01 | - |
| Homoeriodictyol-Natriumsalz | - | - | 0,005 | - | 0,005 | - | 0,005 |
| trans-Pellitorin | - | - | - | - | - | 0,00003 | 0,00003 |
| Verbindung 1 | 0,0002 | 0,0001 | - | 0,0005 | 0,0001 | 0,0002 | 0,0001 |
| Verbindung 4 | - | 0,0001 | 0,0002 | - | - | - | 0,0001 |
| Wasser | ad 100 | | | | | | |

### Anwendungsbeispiel 2: Verwendung in einem Kaugummi

Teile I bis IV werden gemischt und intensiv geknetet. Die Rohmasse kann z.B. in Form von dünnen Streifen zu verzehrfertigen Kaugummis verarbeitet werden.

| Teil | Inhaltsstoff | Einsatz in Gew.-% | | | | |
|---|---|---|---|---|---|---|
| | | A (nicht erfindungsgemäß) | B | C | D | E |
| I | Kaugummibase, Company "Jagum T" | 30,495 | 30,495 | 30,4948 | 30,495 | 30,495 |
| II | Sorbit, pulverisiert | 39,00 | 39,00 | 39,00 | 39,00 | 39,00 |
| | Isomalt^{®} (Palatinit GmbH) | 9,50 | 9,50 | 9,50 | 9,50 | 9,50 |
| | Xylit | 2,00 | 2,00 | 2,00 | 2,00 | 2,00 |
| | Mannit | 3,00 | 3,00 | 3,00 | 3,00 | 3,00 |
| | Aspartam^{®} | 0,10 | 0,10 | 0,10 | 0,10 | 0,10 |
| | Acesulfam^{®} K | 0,10 | 0,10 | 0,10 | 0,10 | 0,10 |
| | Emulgum^{®} (Colloides Naturels, Inc.) | 0,30 | 0,30 | 0,30 | 0,30 | 0,30 |
| III | Sorbitol, 70% | 14,00 | 14,00 | 14,00 | 14,00 | 14,00 |
| | Glycerin | 1,00 | 1,00 | 1,00 | 1,00 | 1,00 |
| IV | Pfefferminzaroma | 0,5 | 0,5 | - | 0,5 | 0,5 |
| | Zimt-Frucht-Aroma | - | - | 0,5 | - | - |
| | trans-Pellitorin | - | - | 0,0002 | - | - |
| | Nonivamid | - | - | - | 0,00001 | 0,00001 |
| | Verbindung 1 | 0,0050 | - | 0,0025 | 0,0025 | - |
| | Verbindung 7 | - | 0,0050 | 0,0025 | 0,0025 | 0,0050 |

### Anwendungsbeispiel 3: Verwendung in Hartkaramellen

Für die zuckerhaltigen Hartkaramellen werden Zucker, Glucosesirup und Wasser gemischt, aufgeschmolzen und bis auf 140°C eingekocht. Die Aromen und die restlichen Bestandteile (Farbe, ggf. Säuremischung) werden zugegeben und nach dem Durchmischen in Formen gegossen. Nach dem Erstarren aus den Formen entfernt und anschließend einzeln verpackt.

Für die zuckerfreien Hartkaramellen werden Palatinit und Wasser gemischt, aufgeschmolzen und bis auf 160°C eingekocht. Die Aromen und die restlichen Bestandteile (Farbe, ggf. Säuremischung) werden zugegeben und nach dem Durchmischen in Formen gegossen. Nach dem Erstarren aus den Formen entfernt und anschließend einzeln verpackt.

| Inhaltsstoff | Gehalt (Gew.-%) | | | | |
|---|---|---|---|---|---|
| | A | B | C | D | E |
| Zucker | 42,5 | - | - | - | - |
| Palatinit, Typ M | - | 75 | 75 | 75,00 | 75,00 |
| Glucosesirup DE40 | 42,5 | - | - | - | - |
| gepufferte Säuremischung Milchsäure/Citronensäure | - | 2 | 2 | - | - |
| Farbstoff gelb | - | 0,01 | - | - | - |
| Farbstoff rot | - | - | 0,01 | - | - |
| Farbstoff blau | 0,01 | - | - | 0,01 | 0,01 |
| Pfefferminzaroma | 0,1 | - | - | 0,1 | 0,1 |
| Zitronenaroma | - | 0,1 | - | - | - |
| Rotfruchtaroma | - | - | 0,1 | - | - |
| Rebaudiosid A 98 % | - | 0,040 | - | 0,040 | 0,040 |
| Balansin A nach WO 2012 164,062 | - | 0,005 | 0,010 | 0,005 | - |
| Hesperetin | - | 0,001 | - | 0,001 | 0,001 |
| Phloretin | - | 0,002 | - | - | - |
| trans-Pellitorin | - | - | 0,00002 | 0,00002 | - |
| Nonivamid | - | - | 0,000005 | - | 0,00001 |
| Verbindung 5 | 0,0002 | 0,0001 | - | 0,0001 | 0,0002 |
| Verbindung 8 | - | 0,0001 | 0,0002 | 0,0001 | - |
| Wasser | ad 100 | ad 100 | ad 100 | ad 100 | ad 100 |

### Anwendungsbeispiel 4: Fettarme Joghurts

Die Inhaltsstoffe wurden gemischt und auf 5°C gekühlt.

| | Zubereitung (Angaben als Gew.-%) | | | |
|---|---|---|---|---|
| Inhaltsstoff | A | B | C | D |
| Sucrose | 10 | 8 | 6 | - |
| Rebaudiosid A 98 % | - | - | - | 0,050 |
| Extrakt aus Rubus suavissimus, enthaltend 20 Gew.-% Rubusosid bezogen auf das Gesamtgewicht des Extraktes, z.B. von PlantExtrakt | - | 0,010 | 0,010 | - |
| Erdbeeraroma | 0,1 | 0,1 | 0,1 | 0,1 |
| Hesperetin | - | 0,001 | 0,001 | 0,002 |
| Phloretin | - | - | 0,002 | 0,002 |
| Homoeriodictyol-Natriumsalz | - | - | - | 0,005 |
| Verbindung 10 | 0,0005 | 0,00025 | - | 0,0002 |
| Verbindung 11 | | 0,00025 | 0,0004 | 0,0002 |
| Nonivamid | - | - | - | 0,00001 |
| Joghurt, 0,1 % Fett | ad 100 | | | |

### Anwendungsbeispiel 5: Fruchtgummis

Saccharose, Glucosesirup, Iso Syrup, Polydextrose und Wasser werden gemischt, aufgeschmolzen und bis auf 115°C eingekocht. In die heiße Masse wird die gequollene und aufgeschmolzene Gelatine-Lösung gegeben. Anschließend werden die Aromen und die restlichen Bestandteile (Farbe, ggf. Säuremischung) zugegeben und nach dem Durchmischen in Formen gegossen und trocknen lassen.

| Inhaltsstoff | Zubereitung (Angaben als Gew.-%) | | | |
|---|---|---|---|---|
| | A | B | C | D |
| Saccharose | 34,50 | 8,20 | 8,20 | 34,50 |
| Glucosesirup, DE 40 | 32 | 30 | 30 | 32 |
| Iso Syrup C* Tru Sweet 01750 (Cerestar GmbH) | 1,50 | 2,10 | 2,10 | 1,50 |
| Gelatine 240 Bloom 1:2 in Wasser gequollen und aufgelöst | 19,5 | 21 | 21 | 19,5 |
| Polydextrose (Litesse^{®} Ultra, Danisco Cultor GmbH) | - | 24,40 | 24,40 | - |
| Gelber und roter Farbstoff | 0,01 | 0,01 | 0,01 | 0,01 |
| Citronensäure | 1 | 1 | 1 | 1 |
| Kirscharoma, enthaltend 1 Gew.-% Hesperetin und 0,3 Gew.-% Phloretin bezogen auf das Aroma | - | 0,10 | 0,10 | - |
| Kirscharoma | 0,1 | - | - | 0,1 |
| trans-Pellitorin | - | - | 0,00002 | - |
| Nonivamid | - | - | 0,000005 | - |
| Verbindung 2 | 0,0002 | 0,0001 | - | 0,0002 |
| Verbindung 6 | - | 0,0001 | 0,0002 | - |
| Wasser | ad 100 | ad 100 | ad 100 | ad 100 |

Polydextrose ist ein nicht süß schmeckendes Polysaccharid mit niedrigem Brennwert.

### Anwendungsbeispiel 6 Fruchtsäfte und Fruchtsaftgetränke

| Inhaltsstoff | Einsatz in Gew.-% | | | | | | |
|---|---|---|---|---|---|---|---|
| Zubereitung | A (nicht erfindungsgemäß) | B | C | D (nicht erfindungsgemäß) | E (nicht erfindungsgemäß) | F (nicht erfindungsgemäß) | G (nicht erfindungsgemäß) |
| Apfelsaft | ad 100 | - | - | - | ad 100 | - | ad 100 |
| Apfelsaftkonzentrat 10fach | - | 10 | - | 10 | - | 10 | - |
| Recovery-Aroma aus Apfelsaftkonzentrat | - | 0,1 | - | 0,1 | - | 0,1 | - |
| Orangensaftkonzentrat 20fach | - | - | 5 | - | - | - | - |
| Recovery-Aroma aus Orangensaftkonzentrat | - | - | 0,05 | - | - | - | - |
| Wasser | - | ad 100 | ad 100 | ad 100 | - | ad 100 | - |
| Ascorbinsäure | 0,05 | 0,05 | 0,05 | - | - | - | - |
| trans-Pellitorin | - | - | - | - | - | 0,00002 | - |
| Nonivamid | - | - | - | - | 0,00000 5 | 0,00000 5 | 0,000005 |
| Verbindung 1 | 0,0002 | 0,0001 | - | 0,0002 | 0,0001 | 0,0002 | 0,0002 |
| Verbindung 9 | - | 0,0001 | 0,0002 | - | - | - | - |

### Anwendungsbeispiel 7: Milchmixgetränk auf Trockenbasis

Die Zutaten werden gemischt und unter Schutzgas oder im Vakuumbeutel abgepackt. Zur Verwendung wird das Pulver in 100 ml Wasser (Zimmertemperatur) aufgerührt und dann direkt verzehrt.

| Inhaltsstoff | Einsatz in Gramm | | | | | | |
|---|---|---|---|---|---|---|---|
| Zubereitung | A | B | C | D | E | F | G |
| Zucker, fein | 5 | 5 | 5 | 5 | 5 | 5 | 5 |
| Nonivamid, 0,1%ig in Alkohol | - | - | 0,03 | 0,03 | - | 0,015 | - |
| trans-Pellitorin, 1 %ig in 1,2-Propylenglycol/Diethylmalonat | - | 0,015 | - | 0,015 | - | - | 0,01 |
| Verbindung 8, 1 %ig in Alkohol | 0,03 | 0,03 | 0,03 | 0,03 | 0,05 | 0,03 | 0,03 |
| Aroma Typ Cappuccino sprühgetrocknet | 0,2 | 0,2 | 0,2 | 0,2 | - | - | - |
| Aroma Typ Erdbeer sprühgetrocknet | - | - | - | - | 0,1 | 0,1 | 0,1 |
| Rote Bete Farbpulver sprühgetrocknet | - | - | - | - | 0,1 | 0,08 | 0,1 |
| Zuckercouleur | 0,15 | 0,15 | 0,15 | 0,15 | - | - | - |
| Xanthan | 0,1 | 0,1 | 0,1 | 0,1 | 0,1 | 0,1 | 0,1 |
| Magermilchpulver | 10 | 10 | 10 | - | 10 | - | - |
| Milchpulver lactosereduziert (Fa. Omira, Ravensburg) | - | - | - | - | - | 10 | 10 |
| Lupinenproteinpulver | - | - | - | 5 | - | - | - |

### Anwendungsbeispiel 8: Molkenproteingetränk

Herstellung eines Frucht-Molkenproteingetränkes nach u.g. Rezeptur mit anschließender Homogenisierung und Pasteurisierung.

| Inhaltsstoff | Einsatz in Gew.-% | |
|---|---|---|
| | A | B |
| Molkenproteinisolat (min. 88% Protein TS) | 8 | 8 |
| Fruchtsaftkonzentrat-Mix (Mango, Banane, Karotte, Orange) | 10 | 10 |
| Zitronensäure 50%ig | 0,2 | 0,2 |
| Pektin | 0,4 | 0,4 |
| Vitamin-Mischung | 0,01 | 0,01 |
| Mineralsalz-Mischung | 1 | 1 |
| Verbindung 2 | 0,0003 | - |
| Verbindung 4 | - | 0,0002 |
| Mango Aroma (Symrise) | 0,05 | 0,05 |
| Trinkwasser | ad 100 | ad 100 |

### Anwendungsbeispiel 9: Proteinriegel

Die Cerealien und das Molkenproteinisolat werden gemischt. Aus den restlichen Zutaten (ohne Aroma) wird ein Sirup gekocht. Am Ende der Kochzeit wird das Aroma zugegeben. Der Sirup wird mit den Cerealien gemischt und zu Riegeln geformt.

| Inhaltsstoff | Einsatz in Gew.-% | | |
|---|---|---|---|
| | A | B | C |
| Haferflocken | 13 | 13 | 13 |
| Reis-Crispies | 14 | 14 | 14 |
| Corn Flakes | 10 | 10 | 10 |
| Molkenproteinisolat (min. 88% Protein TS) | 10 | 10 | 10 |
| Glucose Sirup | 0,1 | 0,1 | 0,1 |
| Saccharose | 12 | 12 | 12 |
| Glycerin | 1 | 1 | 1 |
| Salz | 0,2 | 0,2 | 0,2 |
| Trinkwasser | 13,5 | 13,5 | 13,5 |
| Pflanzenfett | 10 | 10 | 10 |
| Lecithin | 0,2 | 0,2 | 0,2 |
| Bittermasking Aroma (Symrise) | 0,3 | 0,3 | 0,3 |
| Karamel Aroma (Symrise) | 0,1 | 0,1 | 0,1 |
| Verbindung 7 | 0,00025 | 0,0005 | - |
| Verbindung 9 | 0,00025 | - | 0,0004 |

### Anwendungsbeispiel 10: Fertignudelgericht

Alle Inhaltsstoffe der Trockenwürzmischung werden zusammengewogen und homogen gemischt.

| Trockenwürzmischung für Mie Nudeln | |
|---|---|
| Inhaltsstoff | Einsatz in Gew.-% |
| Maltodextrin DE 18-20 | 35 |
| Salz | 30 |
| Hefeextrakt | 10 |
| Knoblauchpulver | 4 |
| Zwiebelpulver | 4 |
| Zucker | 3 |
| Zitronensäure | 1,2 |
| Cayennepfeffer | 0,3 |
| Paprikapulver | 0,5 |
| Trockenaroma Typ Asia | 12 |

6g der o.g. Trockenwürzmischung wird mit den entsprechenden Mengen der Verbindungen 3 bzw. 4 gemischt. Diese Mischung wird zusammen mit den trockenen Mie-Nudeln und den gefriergetrockneten Gemüsestücken in eine Schüssel gegeben. 350mL Wasser werden auf 80°C erhitzt und über die Nudel-Mischung gegossen. Die Mischung umrühren, abdecken und ca. 4 Minuten ziehen lassen, bis die Nudeln gar sind.

| Inhaltsstoff | Einsatz in Gramm [pro Portion] | |
|---|---|---|
| | A (nicht erfindungsgemäß) | B |
| Instant Mie-Nudeln | 70 | 70 |
| gefriergetrocknete Paprikawürfel (rot) | 0,7 | 0,7 |
| gefriergetrocknete Lauchstücke | 0,5 | 0,5 |
| gefriergetrocknete Zwiebelwürfel | 0,4 | 0,4 |
| Trockenwürzmischung für Mie Nudeln | 6 | 6 |
| Verbindung 1 | 0,0007 | - |
| Verbindung 5 | - | 0,0006 |

### Anwendungsbeispiel 11: Tomaten-Instantsuppe

Alle Inhaltsstoffe werden zusammengewogen und homogen gemischt. 30g der Trockenmischung werden in eine Tasse gegeben und mit 250mL kochendem Wasser unter Rühren aufgegossen.

| Inhaltsstoff | Einsatz in Gew.-% | |
|---|---|---|
| | A | B |
| Tomatenpulver | 30 | 30 |
| Maltodextrin DE 18-20 | ad 100 | ad 100 |
| Zucker | 20 | 20 |
| Salz | 10 | 10 |
| Sahnepulver süß | 7 | 7 |
| Stärke | 16 | 16 |
| Olivenöl | 1 | 1 |
| Zwiebelpulver | 1 | 1 |
| Knoblauchpulver | 0,5 | 0,5 |
| gefriergetrocknete Petersilie | 0,3 | 0,3 |
| Äpfelsäure | 0,1 | 0,1 |
| Schwarzer Pfeffer | 0,1 | 0,1 |
| Trockenaroma Typ Tomate | 0,3 | 0,3 |
| Verbindung 5 | 0,002 | - |
| Verbindung 8 | - | 0,003 |

### Anwendungsbeispiel 12: Instantsuppe Typ Orientalische Linsen

Alle Inhaltsstoffe werden zusammengewogen und homogen gemischt. 30g der Trockenmischung werden in eine Tasse gegeben und mit 250mL kochendem Wasser unter Rühren aufgegossen. Vor dem Verzehr einige Minuten quellen lassen und nochmals gut umrühren.

| Inhaltsstoff | Einsatz in Gew.-% | |
|---|---|---|
| | A | B |
| Linsenpulver, rot | 28 | 28 |
| Maltodextrin DE 18-20 | ad 100 | ad 100 |
| Stärke | 18 | 18 |
| Fett Pulver (Vana Crema) | 14 | 14 |
| Hafer Fasern | 9 | 9 |
| Hefeextrakt | 7 | 7 |
| Salz | 5,5 | 5,5 |
| Natrium Caseinat | 5 | 5 |
| Zucker | 3 | 3 |
| Xanthan | 1,4 | 1,4 |
| Zwiebelpulver | 0,8 | 0,8 |
| Knoblauchpulver | 0,6 | 0,6 |
| Gewürztrockenaroma Typ Indisch | 6 | 6 |
| Verbindung 7 | 0,002 | - |
| Verbindung 9 | - | 0,003 |

## Patentansprüche

1. Cinnamylalkoholderivat der Formel I oder Mischung aus zwei oder mehr Cinnamylalkoholderivaten der Formel I 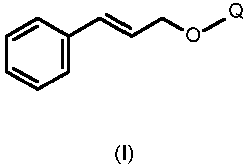
wobei die Doppelbindung eine E- oder Z-Konfiguration aufweisen kann oder eine beliebige Mischung von E- und Z-Konfigurationen vorliegen kann, und wobei Q ein Acylrest der Formel II 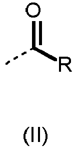
ist, wobei R Wasserstoff oder ein gesättigter oder ungesättigter, linearer oder verzweigter oder cylischer aliphatischer Rest mit 2 bis 6 Kohlenstoffatomen oder Phenyl oder Phenylmethyl ist,
zur Anwendung in einem therapeutischen Verfahren (a) zur Reduzierung des Appetits und/oder (d) zur Reduzierung des Körpergewichts und/oder (e) zur Stimmungsaufhellung, oder
Cinnamylalkoholderivat der Formel I, wobei Q ein Acylrest der Formel II und R Methyl ist,
zur Anwendung in einem therapeutischen Verfahren (a) zur Reduzierung des Appetits und/oder (d) zur Reduzierung des Körpergewichts.

2. Cinnamylalkoholderivat der Formel I oder Mischung aus zwei oder mehr Cinnamylalkoholderivaten der Formel I zur Anwendung nach Anspruch 1,
wobei R ausgewählt ist aus der Gruppe bestehend aus Wasserstoff, Methyl, Ethyl, Propyl, 2-Propyl, 1-Butyl, 2-Butyl, 2-Methyl-1-propyl, 1-Pentyl, 2-Pentyl, 3-Pentyl, 2-Methyl-1-butyl, 2-Methyl-2-butyl, 2-Methyl-3-butyl, 3-Methyl-1-butyl, 1-Hexyl, 2-Hexyl, 3-Hexyl, 2-Methyl-1-pentyl, 2-Methyl-2-pentyl, 2-Methyl-3-pentyl, 2-Methyl-4-pentyl, 3-Methyl-1-pentyl, 3-Methyl-2-pentyl, 3-Methyl-3-pentyl, Vinyl, E- und Z- 1-Propen-1-yl, 2-Propen-1-yl, E- und Z- 2-Buten-2-yl, 3-Buten-2-yl, E- und Z-Prenyl, E- und Z-Isoprenyl, Phenyl und Phenylmethyl.

3. Cinnamylalkoholderivat der Formel I oder Mischung aus zwei oder mehr Cinnamylalkoholderivaten der Formel I zur Anwendung nach Anspruch 1 oder 2, wobei das oder die Cinnamylalkoholderivat(e) ausgewählt ist oder sind aus der Gruppe bestehend aus:

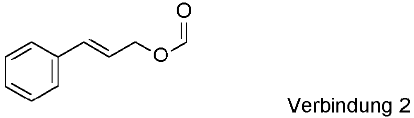

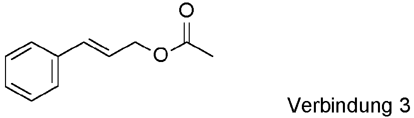

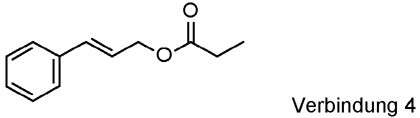

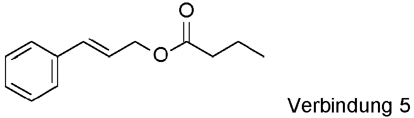

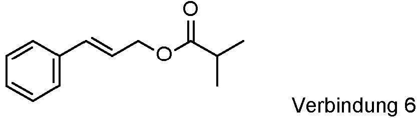

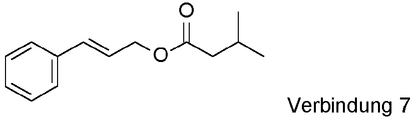

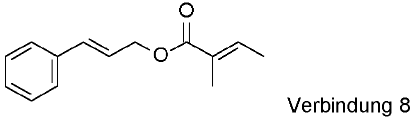

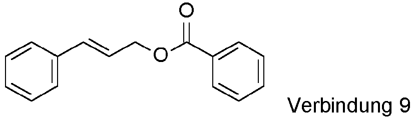

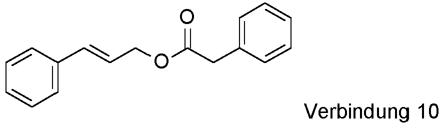

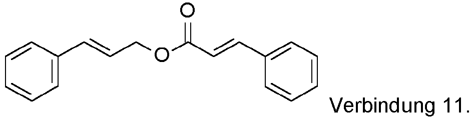

4. Cinnamylalkoholderivat der Formel I oder Mischung aus zwei oder mehr Cinnamylalkoholderivaten der Formel I zur Anwendung nach einem der vorhergehenden Ansprüche, wobei das bzw. die Cinnamylalkoholderivat(e) der Formel I (jeweils) zu mehr als 50 %, bevorzugt zu mehr als 90 %, besonders bevorzugt zu mehr als 95 % in der E-Konfiguration vorliegen.

5. Zubereitung umfassend ein Cinnamylalkoholderivat der Formel I oder eine Mischung aus zwei oder mehr Cinnamylalkoholderivaten der Formel I wie in einem der vorhergehenden Ansprüche definiert zur Anwendung in einem therapeutischen Verfahren wie in Anspruch 1 definiert.

6. Zubereitung nach Anspruch 5 zur Anwendung in einem therapeutischen Verfahren wie in Anspruch 1 definiert, wobei die Gesamtkonzentration der Cinnamylalkoholderivate der Formel I in der Zubereitung bei 20 mg/kg oder weniger, bevorzugt bei 5 mg/kg oder weniger, besonders bevorzugt bei 2 mg/kg oder weniger liegt, jeweils bezogen auf die Gesamtmasse der Zubereitung.

7. Zubereitung nach Anspruch 5 oder 6 zur Anwendung in einem therapeutischen Verfahren wie in Anspruch 1 definiert, wobei die Zubereitung mindestens einen festen Trägerstoff und vorzugsweise mindestens einen weiteren Aromastoff umfasst.

8. Zubereitung nach einem der Ansprüche 5 bis 7 zur Anwendung in einem therapeutischen Verfahren wie in Anspruch 1 definiert, wobei die Zubereitung zusätzlich Nonivamid enthält.

9. Zubereitung nach einem der Ansprüche 5 bis 8 zur Anwendung in einem therapeutischen Verfahren wie in Anspruch 1 definiert, wobei die Cinnamylalkoholderivate der Formel I in einer Konzentration von 0.0001 mg/kg oder mehr, vorzugsweise 0.001 mg/kg oder mehr, besonders bevorzugt 0.005 mg/kg oder mehr vorliegen, jeweils bezogen auf die Gesamtmasse der Zubereitung.

10. Zubereitung nach einem der Ansprüche 5 bis 9 zur Anwendung in einem therapeutischen Verfahren wie in Anspruch 1 definiert, wobei die Zubereitung zusätzlich essbare tierische oder vegane Proteine in Form von Isolaten, Fraktionen, partiellen oder vollständigen Hydrolysaten oder durch physikalisch-chemische Prozesse oder fermentative oder enzymatische Behandlung der Proteine hergestellte Zwischenprodukte und optional einen oder mehrere weitere, die Sättigung beeinflussenden Zusatz- oder Aromastoff, vorzugsweise nicht-verdauliche Kohlenhydrate wie Pektine, beta-Glucane enthält.

11. Zubereitung nach einem der Ansprüche 5 bis 10 zur Anwendung in einem therapeutischen Verfahren wie in Anspruch 1 definiert, wobei die Zubereitung ein oral konsumierbares Produkt ist.

12. Nicht-therapeutische Verwendung eines oder einer Mischung von zwei oder mehr Cinnamylalkoholderivats/en der Formel I 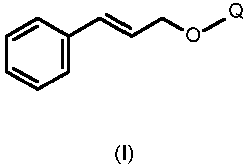
wobei die Doppelbindung eine E- oder Z-Konfiguration aufweisen kann oder eine beliebige Mischung von E- und Z-Konfigurationen vorliegen kann, und wobei Q ein Acylrest der Formel II 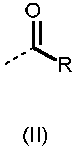
ist, wobei R Wasserstoff oder ein gesättigter oder ungesättigter, linearer oder verzweigter oder cylischer aliphatischer Rest mit 2 bis 6 Kohlenstoffatomen oder Phenyl oder Phenylmethyl ist als (a) Mittel zur Reduzierung des Appetits und/oder (b) Mittel zur Vermittlung eines Gefühls von Sättigung und/oder als (c) Mittel zur Reduzierung der Energieaufnahme und/oder (d) Mittel zur Reduzierung des Körpergewichts und/oder als (e) Stimmungsaufheller oder
Cinnamylalkoholderivat der Formel I, wobei Q ein Acylrest der Formel II und R Methyl ist, als (a) Mittel zur Reduzierung des Appetits und/oder (b) Mittel zur Vermittlung eines Gefühls von Sättigung und/oder als (c) Mittel zur Reduzierung der Energieaufnahme und/oder (d) Mittel zur Reduzierung des Körpergewichts.

13. Nicht-therapeutisches Verfahren zur (i) Reduzierung des Appetits und/oder (ii) Vermittlung eines Gefühls von Sättigung und/oder (iii) Reduzierung der Energieaufnahme und/oder (iv) Reduzierung des Körpergewichts und/oder (v) Aufhellung der Stimmung umfassend den Schritt
- Verabreichen einer (a) den Appetit reduzierenden und/oder (b) ein Gefühl von Sättigung bewirkenden und/oder (c) einer stimmungsaufhellenden Menge von einem oder einer Mischung aus zwei oder mehr Cinnamylalkoholderivaten der Formel I 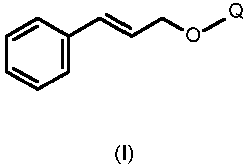 wobei die Doppelbindung eine E- oder Z-Konfiguration aufweisen kann oder eine beliebige Mischung von E- und Z-Konfigurationen vorliegen kann, und wobei Q ein Acylrest der Formel II 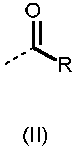 ist, wobei R Wasserstoff oder ein gesättigter oder ungesättigter, linearer oder verzweigter oder cylischer aliphatischer Rest mit 2 bis 6 Kohlenstoffatomen oder Phenyl oder Phenylmethyl, ist an ein Individuum, oder
- Verabreichen einer (a) den Appetit reduzierenden und/oder (b) ein Gefühl von Sättigung bewirkenden Menge von einem Cinnamylalkoholderivat der Formel I, wobei Q ein Acylrest der Formel II und R Methyl ist, an ein Individuum.

## Claims

1. Cinnamyl alcohol derivative of Formula I or a mixture of two or more cinnamyl alcohol derivatives of Formula I 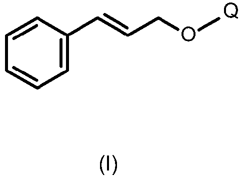
wherein the double bond may have an E- or Z-configuration, or any mixture of E- and Z-configurations may be present, and wherein Q is an acyl residue of Formula II 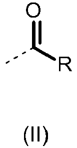
wherein R is hydrogen or a saturated or unsaturated, linear, branched, or cyclic aliphatic residue with 2 to 6 carbon atoms, or phenyl, or phenylmethyl,
for use in a therapeutic method (a) for reducing appetite and/or (d) for reducing body weight and/or (e) for mood elevation, or
cinnamyl alcohol derivative of Formula I, wherein Q is an acyl residue of Formula II and R is methyl,
for use in a therapeutic method (a) for reducing appetite and/or (d) for reducing body weight.

2. Cinnamyl alcohol derivative of Formula I or a mixture of two or more cinnamyl alcohol derivatives of Formula I for use according to claim 1,
wherein R is selected from the group consisting of hydrogen, methyl, ethyl, propyl, 2-propyl, 1-butyl, 2-butyl, 2-methyl-1-propyl, 1-pentyl, 2-pentyl, 3-pentyl, 2-methyl-1-butyl, 2-methyl-2-butyl, 2-methyl-3-butyl, 3-methyl-1-butyl, 1-hexyl, 2-hexyl, 3-hexyl, 2-methyl-1-pentyl, 2-methyl-2-pentyl, 2-methyl-3-pentyl, 2-methyl-4-pentyl, 3-methyl-1-pentyl, 3-methyl-2-pentyl, 3-methyl-3-pentyl, vinyl, E- and Z-1-propen-1-yl, 2-propen-1-yl, E- and Z-2-buten-2-yl, 3-buten-2-yl, E- and Z-prenyl, E- and Z-isoprenyl, phenyl and phenylmethyl.

3. Cinnamyl alcohol derivative of Formula I or a mixture of two or more cinnamyl alcohol derivatives of Formula I for use according to claim 1 or 2, wherein the cinnamyl alcohol derivative(s) is/are selected from the group consisting of:

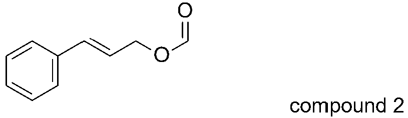

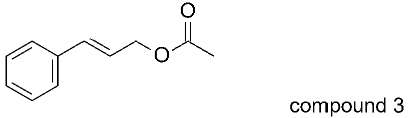

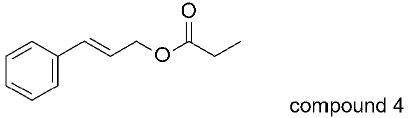

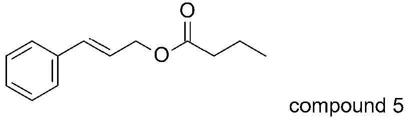

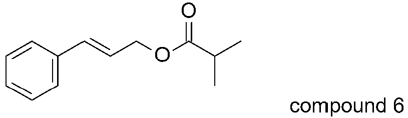

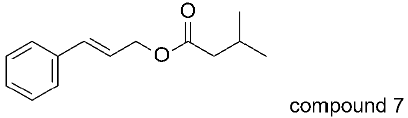

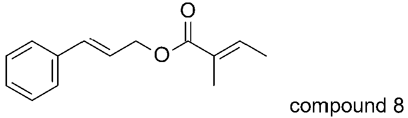

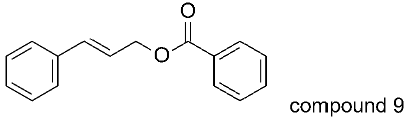

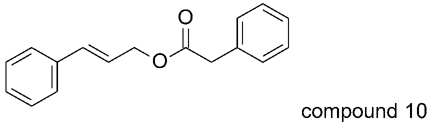

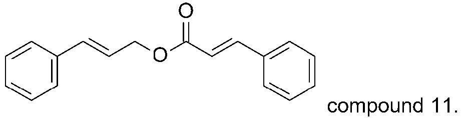

4. Cinnamyl alcohol derivative of Formula I or a mixture of two or more cinnamyl alcohol derivatives of Formula I for use according to any of the preceding claims, wherein the cinnamyl alcohol derivative(s) of Formula I is/are present (each) in more than 50%, preferably more than 90%, particularly preferably more than 95% in the E-configuration.

5. Preparation comprising a cinnamyl alcohol derivative of Formula I or a mixture of two or more cinnamyl alcohol derivatives of Formula I as defined in any of the preceding claims, for use in a therapeutic method as defined in claim 1.

6. Preparation according to claim 5 for use in a therapeutic method as defined in claim 1, wherein the total concentration of the cinnamyl alcohol derivatives of Formula I in the preparation is 20 mg/kg or less, preferably 5 mg/kg or less, particularly preferably 2 mg/kg or less, each relative to the total mass of the preparation.

7. Preparation according to claim 5 or 6 for use in a therapeutic method as defined in claim 1, wherein the preparation comprises at least one solid carrier and preferably at least one further flavoring substance.

8. Preparation according to any of claims 5 to 7 for use in a therapeutic method as defined in claim 1, wherein the preparation additionally contains nonivamide.

9. Preparation according to any of claims 5 to 8 for use in a therapeutic method as defined in claim 1, wherein the cinnamyl alcohol derivatives of Formula I are present at a concentration of 0.0001 mg/kg or more, preferably 0.001 mg/kg or more, particularly preferably 0.005 mg/kg or more, each relative to the total mass of the preparation.

10. Preparation according to any of claims 5 to 9 for use in a therapeutic method as defined in claim 1, wherein the preparation additionally contains edible animal or vegan proteins in the form of isolates, fractions, partial or complete hydrolysates, or intermediates produced by physicochemical processes or fermentative or enzymatic treatment of the proteins, and optionally one or more additional additives or flavoring substances that influence satiety, preferably non-digestible carbohydrates such as pectins and beta-glucans.

11. Preparation according to any of claims 5 to 10 for use in a therapeutic method as defined in claim 1, wherein the preparation is an orally consumable product.

12. Non-therapeutic use of one or a mixture of two or more cinnamyl alcohol derivatives of Formula I 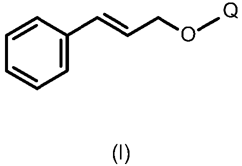
wherein the double bond may have an E- or Z-configuration, or any mixture of E- and Z-configurations may be present, and wherein Q is an acyl residue of Formula II 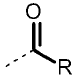 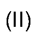
wherein R is hydrogen or a saturated or unsaturated, linear, branched, or cyclic aliphatic residue with 2 to 6 carbon atoms, or phenyl, or phenylmethyl,
as (a) an agent for reducing appetite and/or (b) an agent for conveying a feeling of satiety and/or (c) an agent for reducing energy intake and/or (d) an agent for reducing body weight and/or (e) a mood elevator, or
cinnamyl alcohol derivative of Formula **I,** wherein Q is an acyl residue of Formula II and R is methyl, as (a) an agent for reducing appetite and/or (b) an agent for conveying a feeling of satiety and/or (c) an agent for reducing energy intake and/or (d) an agent for reducing body weight.

13. Non-therapeutic method for (i) reducing appetite and/or (ii) conveying a feeling of satiety and/or (iii) reducing energy intake and/or (iv) reducing body weight and/or (v) elevating mood, comprising the step of:
- administering an (a) appetite-reducing and/or (b) satiety-inducing and/or (c) mood-elevating amount of one or a mixture of two or more cinnamyl alcohol derivatives of Formula I 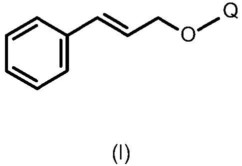 wherein the double bond may have an E- or Z-configuration, or any mixture of E- and Z-configurations may be present, and wherein Q is an acyl residue of Formula II 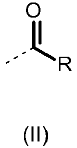 wherein R is hydrogen or a saturated or unsaturated, linear, branched, or cyclic aliphatic residue with 2 to 6 carbon atoms, or phenyl or phenylmethyl, to an individual, or
- administering an (a) appetite-reducing and/or (b) satiety-inducing amount of a cinnamyl alcohol derivative of Formula I, wherein Q is an acyl residue of Formula II and R is methyl, to an individual.

## Revendications

1. Dérivé d'alcool cinnamique de formule I ou mélange de deux ou de plusieurs dérivés d'alcool cinnamique de formule I 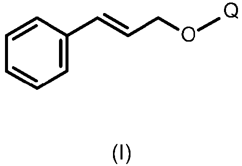
dans laquelle la double liaison peut présenter une configuration E ou Z ou un mélange quelconque de configurations E et Z peut être présent, et dans laquelle Q est un groupe acyle de formule II 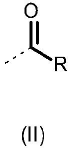
dans laquelle R est de l'hydrogène ou un groupe aliphatique saturé ou insaturé, linéaire ou ramifié ou cyclique, comprenant de 2 à 6 atomes de carbone, ou du phényle ou du phénylméthyle,
destiné à être utilisé dans un procédé thérapeutique (a) afin de réduire l'appétit et/ou (d) afin de réduire le poids corporel et/ou (e) afin d'améliorer l'humeur, ou
Dérivé d'alcool cinnamique de formule I, dans lequel Q est un groupe acyle de formule II et R est du méthyle,
destiné à être utilisé dans un procédé thérapeutique (a) afin de réduire l'appétit et/ou (d) afin de réduire le poids corporel.

2. Dérivé d'alcool cinnamique de formule I ou mélange de deux ou de plusieurs dérivés d'alcool cinnamique de formule I, destiné à être utilisé selon la revendication 1,
dans lequel R est choisi dans le groupe constitué par: l'hydrogène, le méthyle, l'éthyle, le propyle, le 2-propyle, le 1-butyle, le 2-butyle, le 2-méthyl-1-propyle, le 1-pentyle, le 2-pentyle, le 3-pentyle, le 2-méthyl-1-butyle, le 2-méthyl-2-butyle, le 2-méthyl-3-butyle, le 3-méthyl-1-butyle, le 1-hexyle, le 2-hexyle, le 3-hexyle, le 2-méthyl-1-pentyle, le 2-méthyl-2-pentyle, le 2-méthyl-3-pentyle, le 2-méthyl-4-pentyle, le 3-méthyl-1-pentyle, le 3-méthyl-2-pentyle, le 3-méthyl-3-pentyle, le vinyle, le E- et Z-1-propén-1-yle, le 2-propén-1-yle, le E- et Z-2-butén-2-yle, le 3-butén-2-yle, le E- et Z-prényle, le E- et Z-isoprényle, le phényle et le phénylméthyle.

3. Dérivé d'alcool cinnamique de formule I ou mélange de deux ou de plusieurs dérivés d'alcool cinnamique de formule I, destiné à être utilisé selon la revendication 1 ou 2, le ou les dérivé(s) d'alcool cinnamique étant choisis dans le groupe constitué par:

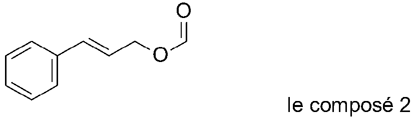

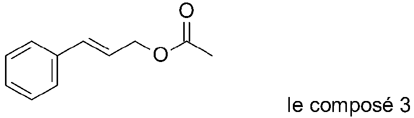

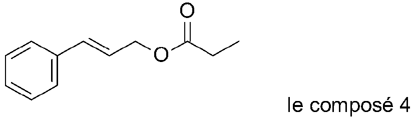

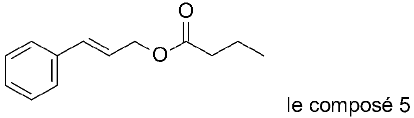

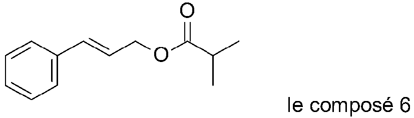

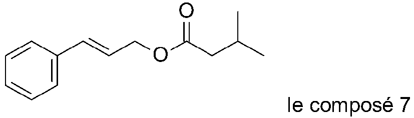

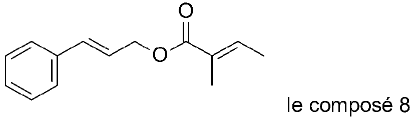

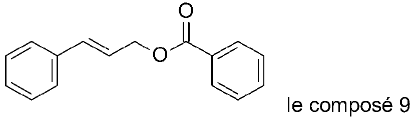

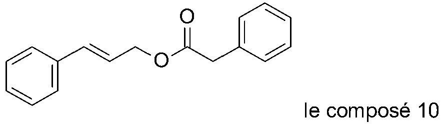

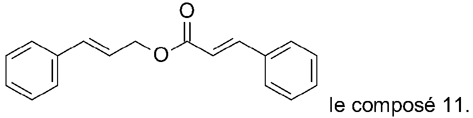

4. Dérivé d'alcool cinnamique de formule I ou mélange de deux ou de plusieurs dérivés d'alcool cinnamique de formule I, destiné à être utilisé selon l'une quelconque des revendications précédentes, dans lequel le ou bien les dérivé(s) d'alcool cinnamique de formule I sont (chacun) présents sous la configuration E à plus de 50 %, de préférence à plus de 90 %, de manière particulièrement préférée à plus de 95 %.

5. Préparation comprenant un dérivé d'alcool cinnamique de formule I ou un mélange de deux ou de plusieurs dérivés d'alcool cinnamique de formule I, tel que défini dans l'une quelconque des revendications précédentes, destinée à être utilisée dans un procédé thérapeutique tel que défini dans la revendication 1.

6. Préparation selon la revendication 5, destinée à être utilisée dans un procédé thérapeutique tel que défini dans la revendication 1, dans laquelle la concentration totale des dérivés d'alcool cinnamique de formule I dans la préparation est de 20 mg/kg ou moins, de préférence de 5 mg/kg ou moins, de manière particulièrement préférée de 2 mg/kg ou moins, respectivement par rapport à la masse totale de la préparation.

7. Préparation selon la revendication 5 ou 6, destinée à être utilisée dans un procédé thérapeutique tel que défini dans la revendication 1, dans laquelle la préparation comprend au moins un support solide et de préférence au moins une autre substance aroma-tisante.

8. Préparation selon l'une quelconque des revendications 5 à 7, destinée à être utilisée dans un procédé thérapeutique tel que défini dans la revendication 1, dans laquelle la préparation contient en outre du nonivamide.

9. Préparation selon l'une quelconque des revendications 5 à 8, destinée à être utilisée dans un procédé thérapeutique tel que défini dans la revendication 1, dans laquelle les dérivés d'alcool cinnamique de formule I sont présents à une concentration de 0,0001 mg/kg ou plus, de préférence de 0,001 mg/kg ou plus, de manière particulièrement préférée de 0,005 mg/kg ou plus, respectivement par rapport à la masse totale de la préparation.

10. Préparation selon l'une quelconque des revendications 5 à 9, destinée à être utilisée dans un procédé thérapeutique tel que défini dans la revendication 1, dans laquelle la préparation comprend en outre des protéines animales ou végétaliennes comestibles sous forme d'isolats, de fractions, d'hydrolysats partiels ou complets, ou de produits intermédiaires obtenus par des processus physico-chimiques ou par traitement fermentatif ou enzymatique des protéines, et en option un ou plusieurs autres additifs ou substances aromatisantes influençant la satiété, de préférence des glucides non digestibles tels que des pectines ou des bêta-glucanes.

11. Préparation selon l'une quelconque des revendications 5 à 10, destinée à être utilisée dans un procédé thérapeutique tel que défini dans la revendication 1, dans laquelle la préparation se présente sous la forme d'un produit consommable par voie orale.

12. Utilisation non thérapeutique d'un dérivé de l'alcool cinnamique ou d'un mélange de deux ou de plusieurs dérivés de l'alcool cinnamique de formule I 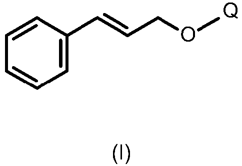 dans laquelle la double liaison peut présenter une configuration E ou Z ou un mélange quelconque de configurations E et Z peut être présent, et dans laquelle Q est un groupe acyle de formule II 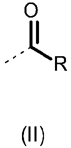
dans laquelle R est de l'hydrogène ou un groupe aliphatique saturé ou insaturé, linéaire ou ramifié ou cyclique, comprenant de 2 à 6 atomes de carbone, ou du phényle ou du phénylméthyle, comme (a) coupe-faim et/ou (b) agent destiné à conférer une sensation de satiété et/ou (c) agent destiné à réduire l'apport énergétique et/ou (d) agent destiné à réduire le poids corporel et/ou comme (e) agent destiné à améliorer l'humeur ou
dérivé d'alcool cinnamique de formule I, dans lequel Q est un groupe acyle de formule II et R est du méthyle, comme (a) coupe-faim et/ou (b) agent destiné à conférer une sensation de satiété et/ou comme (c) agent destiné à réduire l'apport énergétique et/ou (d) agent destiné à réduire le poids corporel.

13. Procédé non thérapeutique destiné à (i) réduire l'appétit et/ou (ii) conférer une sensation de satiété et/ou (iii) réduire l'apport énergétique et/ou (iv) réduire le poids corporel et/ou (v) améliorer l'humeur, comprenant l'étape consistant à
- administrer à un individu une quantité d'un dérivé d'alcool cinnamique ou d'un mélange de deux ou de plusieurs dérivés d'alcool cinnamique de formule I, qui (a) réduit l'appétit et/ou (b) provoque une sensation de satiété et/ou (c) améliore l'humeur 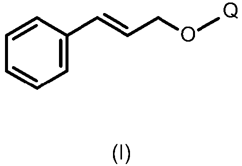 dans laquelle la double liaison peut présenter une configuration E ou Z ou un mélange quelconque de configurations E et Z peut être présent, et dans laquelle Q est un groupe acyle de formule II 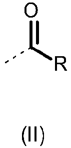 dans laquelle R est de l'hydrogène ou un groupe aliphatique saturé ou insaturé, linéaire ou ramifié ou cyclique, comprenant de 2 à 6 atomes de carbone, ou du phényle ou du phénylméthyle, ou
- administrer à un individu une quantité d'un dérivé d'alcool cinnamique de formule I, qui (a) réduit l'appétit et/ou (b) provoque une sensation de satiété, dans lequel Q est un groupe acyle de formule II et R est du méthyle.
